# EUROPEAN PATENT APPLICATION

(11) **EP 4 715 035 A1**
(43) Date of publication of application: **25.03.2026**
(21) Application number: 24831309.0
(22) Date of filing: 06.02.2024
(51) Int. Cl.: C12M 1/00, C12P 21/08

(54) **BIOREACTOR, BIOREACTOR SYSTEM, AND METHOD FOR OPERATING BIOREACTOR**

(30) Priority: 30.06.2023 JP 2023108838
(71) Applicant: Mitsubishi Heavy Industries, Ltd., Tokyo 100-8332 (JP)
(72) Inventor: HARA, Tomohiro, Tokyo 100-8332 (JP); SAKATA, Nobuyasu, Tokyo 100-8332 (JP); ENDO, Akihisa, Tokyo 100-8332 (JP); HIBINO, Yuhei, Kitakyushu-shi, Fukuoka 803-0814 (JP); ASAI, Yuki, Tokyo 100-8332 (JP); OKADA, Koichiro, Kobe-shi, Hyogo 652-8585 (JP); OGAWA, Naoki, Tokyo 100-8332 (JP)
(74) Representative: Studio Torta S.p.A.
(86) International application number: PCT/JP2024/003883
(87) International publication number: WO 2025/004428

(57) **Abstract**

A bioreactor according to the present disclosure comprises: an outer cylinder which is centered on an axis line extending in the vertical direction; an inner cylinder which is provided inside the outer cylinder and centered on the axis line; a rotary cylinder which is disposed between the outer cylinder and the inner cylinder, defines a first pool between the inner cylinder and the rotary cylinder, and is rotatable about the axis line; a drive unit for rotationally driving the rotary cylinder around the axis line; a first supply port through which a liquid is supplied to the first pool; and a second supply port through which a liquid is supplied to the second pool.

## Description

### Technical Field

The present disclosure relates to a bioreactor, a bioreactor system, and a method for operating a bioreactor.

Priority is claimed on Japanese Patent Application No. 2023-108838, filed June 30, 2023, the content of which is incorporated herein by reference.

### Background Art

For example, PTL 1 discloses a bioreactor for culturing cells. The bioreactor has a double-cylinder structure including a rotating cylinder and an outer cylinder. A pool that accommodates a culture solution is partitioned between the rotating cylinder and the outer cylinder. Taylor vortices are formed in the culture solution accommodated in the pool by rotating the rotating cylinder. The culture solution is stirred by the Taylor vortices to promote cell culture.

### Citation List

### Patent Literature

[PTL 1] Japanese Unexamined Patent Application Publication No. H8-308560

### Summary of Invention

### Technical Problem

Meanwhile, environmental conditions suitable for culturing vary depending on a growth phase of the cells to be cultured. In particular, it is preferable that a culture medium that is a growth environment of the cells changes a component depending on the growth phase.

However, in a case where the bioreactor disclosed in PTL 1 is used, it is necessary to install a plurality of different bioreactors according to a growth stage of the cells, which increases an installation space.

The present disclosure provides a bioreactor, a bioreactor system, and a method for operating a bioreactor, which can perform culture according to a growth phase of cells with a compact configuration.

### Solution to Problem

A bioreactor according to the present disclosure includes an outer cylinder centered on an axis line extending in a vertical direction; an inner cylinder provided inside the outer cylinder around the axis line; a rotating cylinder disposed between the outer cylinder and the inner cylinder to partition a first pool between the rotating cylinder and the inner cylinder, and a second pool between the rotating cylinder and the outer cylinder and rotatable about the axis line; a drive unit configured to rotationally drive the rotating cylinder about the axis line; a first supply port configured to supply liquid to the first pool; and a second supply port configured to supply liquid to the second pool.

A bioreactor system according to the present disclosure includes the bioreactor; and a control device that controls the bioreactor, in which the bioreactor includes a first culture medium supply unit configured to supply, through the first supply port, a first culture medium as the liquid to the first pool, and a second culture medium supply unit configured to supply, through the second supply port, a second culture medium having a component different from the first culture medium as the liquid to the second pool, and the control device includes a medium supply control unit performing a medium supply step in which the first medium is supplied to the first pool by controlling the first medium supply unit, and the second medium is supplied to the second pool by controlling the second medium supply unit.

A method for operating a bioreactor according to the present disclosure includes an outer cylinder centered on an axis line extending in a vertical direction, an inner cylinder provided inside the outer cylinder around the axis line, a rotating cylinder disposed between the outer cylinder and the inner cylinder to partition a first pool between the rotating cylinder and the inner cylinder, and a second pool between the rotating cylinder and the outer cylinder and rotatable about the axis line, and a drive unit configured to rotationally drive the rotating cylinder about the axis line, the method including: a culture medium supply step of supplying a first culture medium to the first pool and supplying a second culture medium having a component different from the first culture medium to the second pool.

### Advantageous Effects of Invention

According to the present disclosure, it is possible to perform culture corresponding to a growth phase of cells in a compact configuration.

### Brief Description of Drawings

FIG. 1 is a schematic longitudinal cross-sectional view showing an overall configuration of a bioreactor system according to a first embodiment of the present disclosure.
FIG. 2 is a cross-sectional view taken along line II-II of FIG. 1.
FIG. 3 is a functional block diagram of a control device of the bioreactor system according to the first embodiment of the present disclosure.
FIG. 4 is a flowchart showing a flow of processing of the control device of the bioreactor system according to the first embodiment of the present disclosure.
FIG. 5 is a flowchart showing a procedure of a culture step according to the first embodiment of the present disclosure.
FIG. 6 is a flowchart showing a procedure of a transferring step according to the first embodiment of the present disclosure.
FIG. 7 is a schematic longitudinal cross-sectional view showing an overall configuration of a bioreactor system according to a second embodiment of the present disclosure.
FIG. 8 is a cross-sectional view taken along line VIII-VIII of FIG. 7.
FIG. 9 is a horizontal cross-sectional view of a rotating cylinder of the bioreactor system according to the second embodiment of the present disclosure, and shows a state in which the rotating cylinder is rotationally driven in a forward direction.
FIG. 10 is a horizontal cross-sectional view of the rotating cylinder of the bioreactor system according to the second embodiment of the present disclosure, and shows a state in which the rotating cylinder is rotationally driven in a reverse direction.
FIG. 11 is a flowchart showing a procedure of the transfer step according to the second embodiment of the present disclosure.
FIG. 12 is a horizontal cross-sectional view of the rotating cylinder of the bioreactor system according to the modification example of the second embodiment of the present disclosure, and shows a state in which the rotating cylinder is rotationally driven in a forward direction.
FIG. 13 is a horizontal cross-sectional view of the rotating cylinder of the bioreactor system according to the modification example of the second embodiment of the present disclosure, and shows a state in which the rotating cylinder is rotationally driven in a reverse direction.
FIG. 14 is a schematic longitudinal cross-sectional view showing an overall configuration of a bioreactor system according to a third embodiment of the present disclosure.
FIG. 15 is a functional block diagram of a control device of the bioreactor system according to the third embodiment of the present disclosure.
FIG. 16 is a flowchart showing a flow of processing of the control device of the bioreactor system according to the third embodiment of the present disclosure.
FIG. 17 is a schematic longitudinal cross-sectional view showing an overall configuration of a bioreactor system according to a fourth embodiment of the present disclosure.
FIG. 18 is an enlarged view of a main part showing a configuration of a seal portion of the bioreactor system according to a fifth embodiment of the present disclosure.
FIG. 19 is an enlarged view of a main part showing a configuration of a seal portion of the bioreactor system according to a first modification example of the fifth embodiment of the present disclosure.
FIG. 20 is an enlarged view of a main part showing a configuration of a seal portion of the bioreactor system according to a second modification example of the fifth embodiment of the present disclosure.
FIG. 21 is an enlarged view of a main part showing a configuration of a low thermal conductivity member of a bioreactor system according to a sixth embodiment of the present disclosure.
FIG. 22 is a hardware configuration diagram of a control device of the bioreactor system according to the present disclosure.

### Description of Embodiments

### <First Embodiment>

### <bioreactor system>

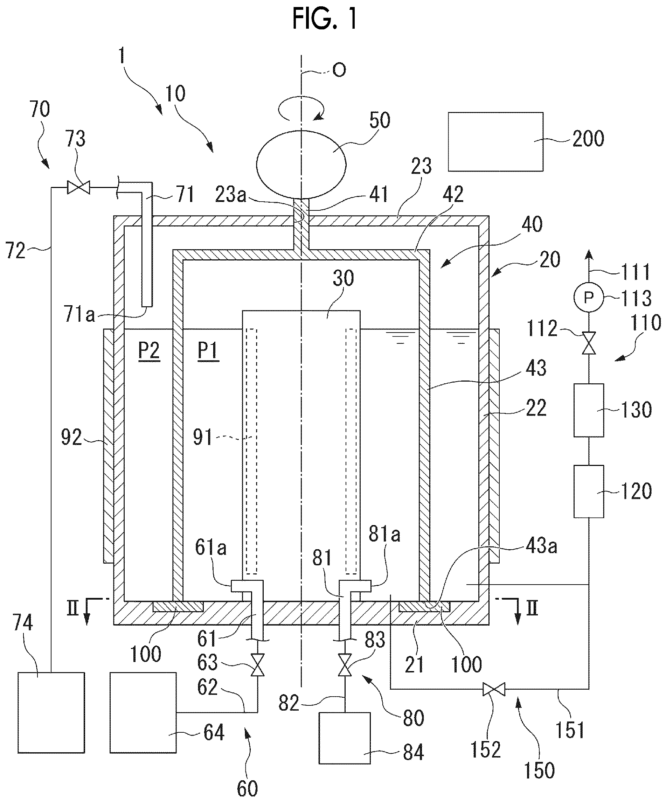

Hereinafter, a first embodiment of the present disclosure will be described in detail with reference to FIGs. 1 to 6.

A bioreactor system 1 shown in FIG. 1 is a system that performs two-stage cell culture such as cell proliferation and antibody production. The bioreactor system 1 manufactures an antibody as a final product. The bioreactor system 1 includes a bioreactor 10 and a control device 200.

### <Bioreactor>

As shown in FIG. 1, the bioreactor 10 includes a casing 20, an inner cylinder 30, a rotating unit 40, a drive unit 50, a first culture medium supply unit 60, a second culture medium supply unit 70, a cell supply unit 80, a first temperature adjusting unit 91, a second temperature adjusting unit 92, an oxygen supply unit 100, a removal unit 110, and a transfer mechanism 150.

### <Casing>

The casing 20 constitutes an outer periphery of the bioreactor 10. The casing 20 has a structure centered on an axis line O extending in a vertical direction. The casing 20 includes a lower bottom portion 21, an outer cylinder 22, and a lid portion 23.

The lower bottom portion 21 is a plate-shaped member that spreads in a horizontal direction and is installed on a floor surface. The lower bottom portion 21 has, for example, a circular shape centered on the axis line O in a plan view.

### <Outer Cylinder>

The outer cylinder 22 is a cylindrical member centered on the axis line O. An outer peripheral surface and an inner peripheral surface of the outer cylinder 22 have a cylindrical surface shape extending in an up-down direction centered on the axis line O. An outer diameter and an inner diameter of the outer cylinder 22 are constant in the up-down direction. The outer cylinder 22 is provided to extend upward from an outer peripheral edge portion of the lower bottom portion 21. That is, the outer cylinder 22 has a configuration in which a lower opening portion is closed by the lower bottom portion 21.

The lid portion 23 closes an upper opening portion of the outer cylinder 22. The lid portion 23 has a bottomed cylindrical shape in which an upper side is closed. A lower end of the lid portion 23 is connected to an upper end of the outer cylinder 22 over an entire periphery. As a result, a sealed space is partitioned and formed in the casing 20, and thus a sterile state is ensured.

A through portion 23a that penetrates the lid portion 23 in the up-down direction is formed in the lid portion 23. The through portion 23a extends in the up-down direction along the axis line O.

### <Inner Cylinder>

The inner cylinder 30 is a cylindrical member centered on the axis line O and is provided inside the outer cylinder 22. An outer peripheral surface of the inner cylinder 30 has a cylindrical surface shape extending in the up-down direction centered on the axis line O. An outer diameter of the inner cylinder 30 is set to be smaller than an inner diameter of the outer cylinder 22 and is constant in the up-down direction. A lower end of the inner cylinder 30 has a circular planar shape that spreads in the horizontal direction. The lower end of the inner cylinder 30 is integrally fixed to the lower bottom portion 21 over an entire surface. An upper end of the inner cylinder 30 has a circular planar shape that extends in the horizontal direction. A height of the upper end of the inner cylinder 30 is a height that can be accommodated in a space in the casing 20.

### <Rotating Unit>

The rotating unit 40 includes an axis portion 41, an upper bottom portion 42, and a rotating cylinder 43.

The axis portion 41 is a shaft-shaped member that extends along the axis line O and is installed to penetrate the through portion of the lid portion 23 in the up-down direction. The axis portion 41 is rotatable about the axis line O, for example, via a bearing (not shown) installed in the through portion.

The upper bottom portion 42 is connected to a lower end of the axis portion 41 in the casing 20. The upper bottom portion 42 is a disk-shaped member that spreads in the horizontal direction and is centered on the axis line O. The upper bottom portion 42 is disposed above the upper end of the inner cylinder 30 at a spacing from the upper end. A center of an upper surface of the upper bottom portion 42 is fixed to the lower end of the axis portion 41. An outer diameter of the upper bottom portion 42 is smaller than the inner diameter of the outer cylinder 22 and larger than the outer diameter of the inner cylinder 30.

### <Rotating Cylinder>

The rotating cylinder 43 is a cylindrical member centered on the axis line O and is disposed between the outer cylinder 22 and the inner cylinder 30 in the casing 20. An outer peripheral surface and an inner peripheral surface of the rotating cylinder 43 have a cylindrical surface shape extending in the up-down direction centered on the axis line O. An outer diameter of the rotating cylinder 43 is set to be smaller than the inner diameter of the outer cylinder 22. An inner diameter of the rotating cylinder 43 is set to be larger than the outer diameter of the inner cylinder 30. An outer diameter and an inner diameter of the rotating cylinder 43 are constant in the up-down direction.

An upper end of the rotating cylinder 43 is integrally connected to an outer peripheral edge portion of the upper bottom portion 42 over an entire circumferential direction. That is, the upper opening of the inner cylinder 30 is closed by the upper bottom portion 42.

A lower end edge portion 43a of the rotating cylinder 43 faces an upper surface of the lower bottom portion 21 through a slight clearance. A dimension of the clearance is slightly larger than a dimension of the entire bioreactor 10. The dimension of the clearance in the up-down direction is set to, for example, 1/1000 to 1/100 of a length of the casing 20 in the up-down direction. The dimension of the clearance is set to, for example, 1 mm to 10 mm.

The rotating cylinder 43 is rotatable about the axis line O together with the axis portion 41 and the upper bottom portion 42. The rotating cylinder 43 can be smoothly rotated without interfering with the lower bottom portion 21 because the clearance is formed between the lower end of the inner cylinder 30 and the lower bottom plate. It should be noted that a configuration in which the lower end of the rotating cylinder 43 is in sliding contact with the upper surface of the upper bottom portion 42 may be adopted.

### <First Pool, Second Pool>

The rotating cylinder 43 partitions two spaces of the first pool P1 and the second pool P2 in the casing 20 to be independent of each other. The first pool P1 and the second pool P2 can accommodate a liquid, respectively. The liquid to be accommodated is a culture solution containing a culture medium and cells.

The first pool P1 is partitioned and formed by the outer peripheral surface of the inner cylinder 30 and the inner peripheral surface of the rotating cylinder 43. The first pool P1 has an annular shape centered on the axis line O and is a space extending in the up-down direction.

The second pool P2 is partitioned and formed by the outer peripheral surface of the rotating cylinder 43 and the inner peripheral surface of the outer cylinder 22. The second pool P2 has an annular shape centered on the axis line O and is a space extending in the up-down direction, and is larger than the first pool P1.

The first pool P1 and the second pool P2 are slightly communicated through the above-described clearance. Since the clearance is slight, the transfer of the culture solution between the first pool P1 and the second pool P2 substantially does not occur.

### <Drive Unit>

The drive unit 50 rotationally drives the rotating unit 40 about the axis line O. The drive unit 50 is provided outside the casing 20. The drive unit 50 is, for example, an electric motor, and an output shaft thereof is connected to the upper end of the axis portion 41 of the rotating unit 40. The rotating unit 40 is rotationally driven at the same rotational speed by the output shaft of the drive unit 50 rotating at a predetermined rotational speed.

It should be noted that the drive unit 50 may be provided inside the casing 20. In this case, for example, the drive unit 50 may be installed at the upper end of the inner cylinder 30, and the rotating unit 40 may be rotationally driven by the drive unit 50.

### <First Culture Medium Supply Unit>

The first culture medium supply unit 60 supplies the first culture medium to the first pool P1. The first culture medium supply unit 60 includes a first supply pipe 61, a first supply line 62, a first supply valve 63, and a first culture medium accommodation portion 64.

The first supply pipe 61 is a pipe inserted from the outside of the casing 20 and has a first supply port 61a that is open toward the first pool P1. The first supply pipe 61 of the present embodiment penetrates the inner cylinder 30 and is provided such that the first supply port 61a is open to a lower portion of the first pool P1.

The first supply line 62 is a pipe that extends outside the casing 20 and is connected to an end portion of the first supply pipe 61 on a side opposite to the first supply port 61a.

The first supply valve 63 is provided on the first supply line 62 and has a configuration in which a flow passage of the first supply line 62 can be opened and closed.

The first culture medium accommodation portion 64 accommodates the first culture medium. The first culture medium is adjusted to have a component suitable for cell proliferation. The first culture medium accommodation portion 64 is connected to an end portion of the first supply line 62 on a side opposite to the first culture medium supply pipe.

The first culture medium supply unit 60 has a configuration in which the first culture medium in the first culture medium accommodation portion 64 is supplied to the first pool P1 via the first supply port 61a through the first supply line 62 and the first supply pipe 61 by changing the first supply valve 63 from a closed state to an open state.

### <Second Culture Medium Supply Unit>

The second culture medium supply unit 70 supplies the second culture medium to the second pool P2. The second culture medium supply unit 70 includes a second supply pipe 71, a second supply line 72, a second supply valve 73, and a second culture medium accommodation portion 74.

The second supply pipe 71 is a pipe inserted from the outside of the casing 20 and has a second supply port 71a that is open toward the second pool P2. The second supply pipe 71 of the present embodiment is provided such that the second supply port 71a is open downward above the second pool P2.

The second supply line 72 is a pipe that extends outside the casing 20 and is connected to an end portion of the second supply pipe 71 on a side opposite to the second supply port 71a.

The second supply valve 73 is provided on the second supply line 72 and has a configuration in which a flow passage of the second supply line 72 can be opened and closed.

The second culture medium accommodation portion 74 accommodates the second culture medium. The second culture medium is adjusted to have a component suitable for cells to produce an antibody while suppressing cell proliferation. The second culture medium accommodation portion 74 is connected to an end portion of the second supply line 72 on a side opposite to the second culture medium supply pipe. The second culture medium supply unit 70 has a configuration in which the second culture medium in the second culture medium accommodation portion 74 is supplied to the second pool P2 via the second supply port 71a through the second supply line 72 and the second supply pipe 71 by changing the second supply valve 73 from a closed state to an open state.

### <Cell Supply Unit>

The cell supply unit 80 supplies cells to the first pool P1. The cell supply unit 80 includes a cell supply pipe 81, a cell supply line 82, a cell supply valve 83, and a cell accommodation portion 84.

The cell supply pipe 81 is a pipe inserted from the outside of the casing 20 and has a cell supply port 81a that is open toward the cell pool. The cell supply pipe 81 of the present embodiment penetrates the inner cylinder 30 and is provided such that the cell supply port 81a is open to a lower portion of the first pool P1.

The cell supply line 82 is a pipe that extends outside the casing 20 and is connected to an end portion of the cell supply pipe 81 on a side opposite to the cell supply port 81a.

The cell supply valve 83 is provided on the cell supply line 82 and has a configuration in which a flow passage of the cell supply line 82 can be opened and closed.

The cell accommodation portion 84 accommodates cells to be cultured. The cell accommodation portion 84 is connected to an end portion of the cell supply line 82 on a side opposite to the cell supply pipe 81.

The cell supply unit 80 has a configuration in which the cells in the cell accommodation portion 84 are supplied to the cell pool via the cell supply port 81a through the cell supply line 82 and the cell supply pipe 81 by changing the cell supply valve 83 from a closed state to an open state.

### <First Temperature Adjusting Unit>

The first temperature adjusting unit 91 adjusts a temperature of the first pool P1. The first temperature adjusting unit 91 sets the temperature of the first pool P1 to a predetermined temperature by heating and cooling the first pool P1.

The first temperature adjusting unit 91 is provided inside the inner cylinder 30. That is, the first temperature adjusting unit 91 is provided inside the inner cylinder 30 over the circumferential direction and the up-down direction, and the temperature of the first pool P1 can be adjusted via the outer peripheral surface of the inner cylinder 30.

### <Second Temperature Adjusting Unit>

The second temperature adjusting unit 92 adjusts a temperature of the second pool P2. The second temperature adjusting unit 92 sets the temperature of the second pool P2 to a predetermined temperature by heating and cooling the second pool P2.

The second temperature adjusting unit 92 is provided to surround the outer cylinder 22 from the outer peripheral side. The second temperature adjusting unit 92 has an annular shape and is provided to extend in the circumferential direction and the up-down direction of the outer cylinder 22. That is, the second temperature adjusting unit 92 can adjust the temperature of the first pool P1 via the inner peripheral surface of the outer cylinder 22.

As the first temperature adjusting unit 91 and the second temperature adjusting unit 92, various temperature control mechanisms such as a heater, a cooler, and a Peltier element can be adopted.

### <Oxygen Supply Unit>

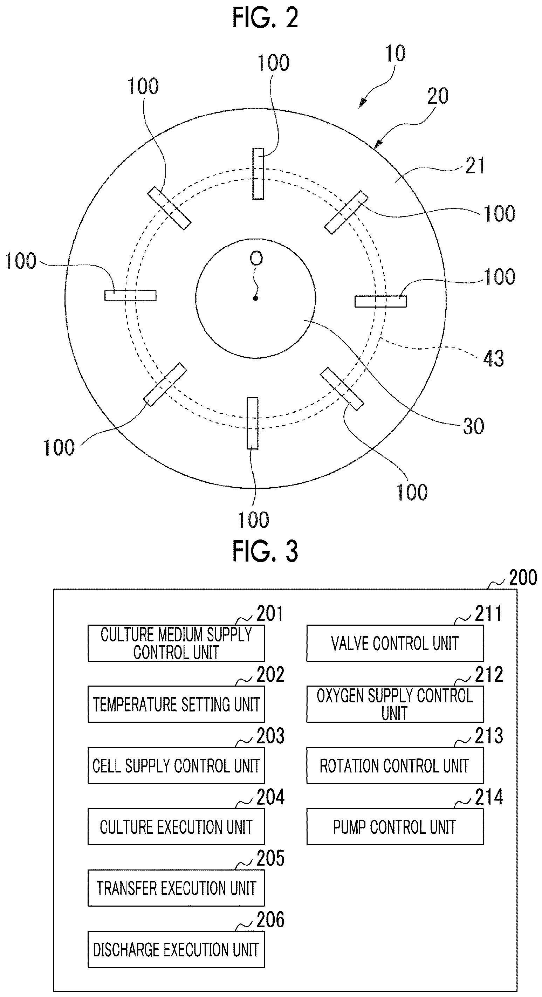

The oxygen supply unit 100 supplies oxygen to the first pool P1 and the second pool P2. As shown in FIGs. 1 and 2, the oxygen supply unit 100 of the present embodiment is disposed on an upper surface of the lower bottom portion 21. A plurality of oxygen supply units 100 are provided at a spacing in the circumferential direction. Each of the oxygen supply units 100 extends in the radial direction of the axis line O and is provided to straddle the first pool P1 and the second pool P2 with the rotating cylinder 43 interposed therebetween in a plan view. Each of the oxygen supply units 100 is a sparger that can release oxygen. The oxygen concentration of the culture solution in the first pool P1 and the second pool P2 is adjusted by releasing oxygen from the sparger.

### <Removal Unit>

The removal unit 110 has a role of removing the liquid of the second pool P2 to the outside. The removal unit 110 includes a discharge line 111, a discharge valve 112, a removal pump 113, a cell acquisition unit 120, and an antibody acquisition unit 130.

The discharge line 111 is a pipe provided outside the casing 20, and one end thereof is connected to the second pool P2.

The discharge valve 112 is provided in the discharge line 111 and is openable and closable.

The removal pump 113 is provided in the discharge line 111 and can pump a fluid from one end of the discharge line 111 toward the other end.

The cell acquisition unit 120 is a filter that can extract cells from the culture solution. The cell acquisition unit 120 is provided on an upstream side of the discharge valve 112 and the removal pump 113 in the discharge line 111.

The antibody acquisition unit 130 is a filter that can extract an antibody produced by the cells from the culture solution. The antibody acquisition unit 130 is provided on the upstream side of the discharge valve 112 and the removal pump 113 in the discharge line 111 and on the downstream side of the cell acquisition unit 120.

With the above-described configuration, the removal unit 110 can discharge the culture solution in the second pool P2 to the outside by closing the discharge valve 112 and operating the removal pump 113.

### <Transfer Mechanism>

The transfer mechanism 150 is a mechanism that can move the liquid of the first pool P1 to the second pool P2. The transfer mechanism 150 of the present embodiment includes a transfer line 151 and a transfer valve 152.

The transfer line 151 is a pipe disposed outside the casing 20, and one end thereof is connected to the first pool P1. The other end of the transfer line 151 is connected to the second pool P2. In the present embodiment, the other end of the transfer line 151 is connected to the upstream side of the cell acquisition unit 120 in the discharge line 111. As a result, the transfer line 151 is connected to the second pool P2 via the discharge line 111. It should be noted that the other end of the transfer line 151 may be directly connected to the second pool P2.

The transfer valve 152 is provided on the discharge line 111 and is openable and closable. In a case where the liquid level of the second pool P2 is lower than the liquid level of the first pool P1, the transfer valve 152 is changed from the closed state to the open state, and the liquid of the first pool P1 moves to the second pool P2 by a head difference.

### <Control Device>

Next, the control device 200 of the present embodiment will be described with reference to FIG. 3. As shown in FIG. 3, the control device 200 includes a culture medium supply control unit 201, a temperature setting unit 202, a cell supply control unit 203, a culture execution unit 204, a transfer execution unit 205, a discharge execution unit 206, a valve control unit 211, an oxygen supply control unit 212, a rotation control unit 213, and a pump control unit 214.

The culture medium supply control unit 201 controls the first culture medium supply unit 60 and the second culture medium supply unit 70 via the valve control unit 211 to supply the first culture medium to the first pool P1 and supply the second culture medium to the second pool P2.

The temperature setting unit 202 controls the first temperature adjusting unit 91 and the second temperature adjusting unit 92 such that the first pool P1 and the second pool P2 have target temperatures, respectively.

The cell supply control unit 203 controls the cell supply unit 80 via the valve control unit 211 to supply the first culture medium to the first pool P1.

The culture execution unit 204 controls the drive unit 50 via the rotation control unit 213 to rotate the rotating unit 40. The culture execution unit 204 controls the oxygen supply unit 100 via the oxygen supply control unit 212 to supply oxygen to the first pool P1 and the second pool P2.

The transfer execution unit 205 controls the transfer mechanism 150 via the valve control unit 211 to move the culture solution from the first pool P1 to the second pool P2.

The discharge execution unit 206 controls the removal unit 110 via the valve control unit 211 and the pump control unit 214 to extract the culture solution from the second pool P2.

The valve control unit 211 controls opening and closing operations of the first supply valve 63, the second supply valve 73, the cell supply valve 83, the transfer valve 152, and the discharge valve 112 in response to instructions from the culture medium supply control unit 201, the cell supply control unit 203, the transfer execution unit 205, and the discharge execution unit 206.

The oxygen supply control unit 212 controls the oxygen supply unit 100 in response to an instruction from the culture execution unit 204.

The rotation control unit 213 controls the drive unit 50 in response to a signal from the culture execution unit 204 to rotationally drive the rotating unit 40.

The pump control unit 214 controls the removal pump 113 in response to an instruction from the discharge execution unit 206.

### <Operation Method of Bioreactor>

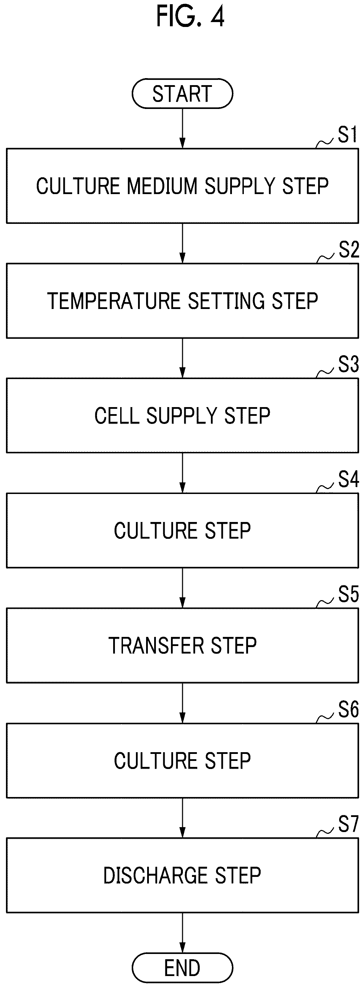

Next, a processing procedure of the control device 200 (operation method of the bioreactor 10) will be described with reference to the flowchart shown in FIG. 4. The control device 200 sequentially executes a culture medium supply step S1, a temperature setting step S2, a cell supply step S3, a culture step S4, a transfer step S5, a culture step S6, and a discharge step S7.

### <Culture Medium Supply Step>

First, the culture medium supply control unit 201 outputs a signal to the valve control unit 211, and the valve control unit 211 changes the first supply valve 63 and the second supply valve 73 from the closed state to the open state. As a result, the first culture medium in the first culture medium accommodation portion 64 is supplied to the first pool P1 via the first supply port 61a of the first supply pipe 61. In addition, the second culture medium in the second culture medium accommodation portion 74 is supplied to the second pool P2 via the second supply port 71a of the second supply pipe 71 (culture medium supply step S1).

In a case where an appropriate amount of the first culture medium and the second culture medium are supplied to the first pool P1 and the second pool P2, the valve control unit 211 changes the first supply valve 63 and the second supply valve 73 from the open state to the closed state. As a result, the supply of the first culture medium and the second culture medium is stopped.

It should be noted that the first culture medium supply unit 60 and the second culture medium supply unit 70 may be controlled such that the liquid level of the first culture medium is higher than the liquid level of the second culture medium in order to smoothly perform the transfer step described below.

### <Temperature Setting Step>

Next, the temperature setting unit 202 adjusts the first culture medium in the first pool P1 and the second culture medium in the second pool P2 to predetermined temperatures by controlling the first temperature adjusting unit 91 and the second temperature adjusting unit 92 (temperature setting step S2). Here, the temperature of the first culture medium is set to a temperature suitable for cell proliferation. That is, the first pool P1 is set to, for example, a temperature of 36°C to 38°C and is preferably set to a temperature of about 37°C. In addition, the temperature of the second culture medium is set to a temperature suitable for cells to produce an antibody while suppressing cell proliferation. That is, the second pool P2 is set to a temperature lower than the first pool P1, for example, 30°C to 33°C, and is preferably set to a temperature of 31°C to 32°C.

### <Cell Supply Step>

Thereafter, the cell supply control unit 203 outputs a signal to the valve control unit 211, and the valve control unit 211 changes the cell supply valve 83 from the closed state to the open state. As a result, the cells in the cell accommodation portion 84 are supplied to the first pool P1 via the cell supply port 81a of the cell supply pipe 81 (cell supply step S3). In a case where an appropriate amount of cells is supplied to the first pool P1, the valve control unit 211 changes the cell supply valve 83 from the open state to the closed state. As a result, the supply of the cells is stopped.

### <Culture Step>

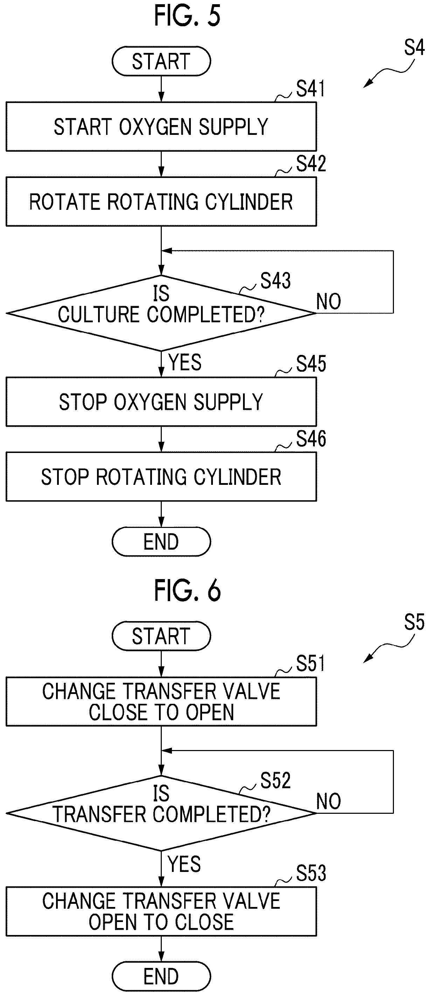

Subsequently, the culture execution unit 204 performs cell culture in the first pool P1 (culture step S4). The culture step S4 is performed in a procedure of the flowchart shown in FIG. 5.

That is, first, the culture execution unit 204 outputs a signal to the oxygen supply control unit 212, and the oxygen supply control unit 212 operates the oxygen supply unit 100. As a result, the oxygen supply to the first pool P1 and the second pool P2 is started (step S41).

Next, the culture execution unit 204 sends a signal to the rotation control unit 213, and the rotation control unit 213 controls the drive unit 50 to rotationally drive the rotating unit 40 (step S42). Here, the rotational speed of the rotating unit 40 is set to a value suitable for forming Taylor vortices in the first pool P1 and the second pool P2. The rotation control unit 213 rotationally drives the rotating cylinder 43 in the forward direction such that the Taylor number in the first pool P1 and the second pool P2 is, for example, 20000 to 30000, taking into consideration the radial dimensions of the first pool P1 and the second pool P2. As a result, turbulence is unlikely to occur in the first pool P1 and the second pool P2, and the first pool P1 is stirred by a stable vortex, and cell proliferation in the first pool P1 is promoted.

Subsequently, the culture execution unit 204 determines whether or not the culture is completed (step S43). In a case where, for example, the cell concentration in the first pool P1 measured by a cell number measuring device does not exceed a predetermined value, the culture execution unit 204 determines that the culture is not completed and continues the forward rotational driving of the rotating cylinder 43 (step S43: No). In a case where, for example, the cell concentration does not exceed the predetermined value, the culture execution unit 204 determines that the culture is not completed (step S43: Yes).

It should be noted that the culture execution unit 204 may determine that the culture is completed at a point in time after the rotating cylinder 43 is rotationally driven and a predetermined time has elapsed.

After the culture is completed, the culture execution unit 204 outputs a signal to the oxygen supply control unit 212, and the oxygen supply control unit 212 stops the oxygen supply by the oxygen supply unit 100 (step S45).

Thereafter, the culture execution unit 204 outputs a signal to the rotation control unit 213, and the rotation control unit 213 stops the rotation of the rotating cylinder 43 (step S46).

As described above, the culture step S4 is completed, and the first culture medium is filled with the proliferated cells.

### <Transfer Step>

Next, as shown in FIG. 4, the transfer execution unit 205 moves the culture solution from the first pool P1 to the second pool P2 after the culture step S4 (transfer step S5). As a result, the cells proliferated in the first pool P1 are supplied to the second culture medium in the second pool P2.

Specifically, as shown in FIG. 6, in the transfer step S5, the transfer execution unit 205 outputs a signal to the valve control unit 211, and the valve control unit 211 changes the transfer valve 152 to the open state (S51). As a result, in a case where the liquid level of the first pool P1 is higher than the liquid level of the second pool P2, the culture solution of the first pool P1 moves to the second pool P2 via the transfer line 151 by the head difference.

In a case where the liquid level of the first pool P1 is not higher than the liquid level of the second pool P2, before the transfer valve 152 is opened, the transfer execution unit 205 may temporarily open the discharge valve 112 by controlling the valve control unit 211 and may temporarily operate the removal pump 113 by controlling the pump control unit 214. As a result, the liquid of the second pool P2 is discharged to the outside, and the liquid level of the first pool P1 can be set to be higher than the liquid level of the second pool P2.

It should be noted that a pump may be provided in the transfer line 151, and the culture solution may be transferred from the first pool P1 to the second pool P2 by driving the pump.

Next, the transfer execution unit 205 determines whether or not the transfer of the culture solution is completed (step S52). In a case where a predetermined time has not elapsed after the transfer valve is opened, the transfer execution unit 205 determines that the transfer is not completed (step S52: No). On the other hand, in a case where a predetermined time has elapsed after the transfer valve is opened, the transfer execution unit 205 determines that the transfer is completed (step S52: Yes).

It should be noted that the transfer execution unit 205 may determine that the transfer of the culture solution is completed in a case where the liquid levels of the first pool P1 and the second pool P2 are equal. In addition, the transfer of the culture solution may be determined to be completed based on the cell concentration detected in the first pool P1 and the second pool P2.

After the transfer of the culture solution is completed, the transfer execution unit 205 outputs a signal to the valve control unit 211, and the valve control unit 211 changes the transfer valve 152 from the open state to the closed state (step S53). As a result, the transfer step S5 is completed.

### <Second Culture Step after Transfer Step>

After the transfer step S5 is completed, as shown in FIG. 4, the same culture step S6 as the culture step S4 is performed. As a result, the culture solution of the second pool P2 is stirred, and the production of the antibody by the cells in the second pool P2 is promoted. It should be noted that in the culture step S6 after the transfer step S5, it is determined that the culture is completed by determining that the production of the antibody is completed at a point in time when the cell concentration in the second pool P2 to be detected starts to decrease (FIG. 5: step S43). In addition, it may be determined that the culture is completed at a point in time after a predetermined time has elapsed.

### <Discharge Step>

After the second culture step S6 after the transfer step S5 is completed, the discharge execution unit 206 discharges the culture solution of the second pool P2 (discharge step S7). That is, the discharge execution unit 206 controls the valve control unit 211 to change the discharge valve 112 from the closed state to the open state and controls the pump control unit 214 to operate the removal pump 113. As a result, the culture solution of the second pool P2 is discharged from the second pool P2 via the discharge line 111 together with the cells and the antibody. In a process of flowing through the discharge line 111, the cells in the culture solution are extracted by the cell acquisition unit 120, and the antibody in the culture solution is extracted by the antibody acquisition unit 130. It should be noted that the extracted cells can be reused by being supplied to the first pool P1 again. In addition, the extracted antibody is used for various applications as a product produced by the present system.

As described above, the operation method of the bioreactor 10 is completed.

### <Operational Effects>

According to the bioreactor 10, the bioreactor system 1, and the operation method of the bioreactor 10 as described above, the cell culture can be performed in each of the first pool P1 and the second pool P2 that are independent of each other. Therefore, for example, the culture of cells in different growth phases such as cell proliferation and antibody production can be performed in one device. Therefore, it is not necessary to use a plurality of devices, and the entire system can be made compact.

In addition, since the culture medium having different components can be supplied to the first pool P1 and the second pool P2, respectively, the culture medium having a component suitable for each of the cell proliferation and the antibody production can be supplied. As a result, the cell culture in each growth phase can be efficiently performed.

Here, in order to stir the culture solution in each of the first pool P1 and the second pool P2, for example, it is considered to rotate each of the inner cylinder 30 and the outer cylinder 22. However, in this case, since the two cylinders need to be rotated, the device configuration is complicated. In addition, in order to rotate the outer cylinder 22, more power is required than in a case of rotating the rotating cylinder 43. In the present embodiment, since the first pool P1 and the second pool P2 can be stirred by rotating only the rotating cylinder 43, the device configuration is not complicated, and an efficient device configuration can be adopted.

In addition, in the present embodiment, since the inner cylinder 30 does not need to be rotated, the first culture medium supply pipe and the cell supply pipe 81 can be installed to penetrate the inner cylinder 30. Therefore, the effective use of the space inside the inner cylinder 30 can be achieved, and the installation space of the entire device can be reduced.

Furthermore, since the first pool P1 and the second pool P2 are temperature-controlled by the first temperature adjusting unit 91 and the second temperature adjusting unit 92, respectively, which can be independently set to a temperature, the first pool P1 and the second pool P2 can be set to temperatures suitable for the respective cultures. As a result, the cell culture in different growth phases can be performed more efficiently.

In addition, the cells proliferated in the first pool P1 can be transferred to the second pool P2 to produce the antibody. That is, since a series of processes from cell proliferation to antibody production can be completed in one device, the antibody can be efficiently produced.

Furthermore, in the present embodiment, since the oxygen supply unit 100 is disposed to straddle the first pool P1 and the second pool P2, oxygen can be efficiently supplied to both the first pool P1 and the second pool P2. As a result, an environment suitable for the cells can be set.

### <Second Embodiment>

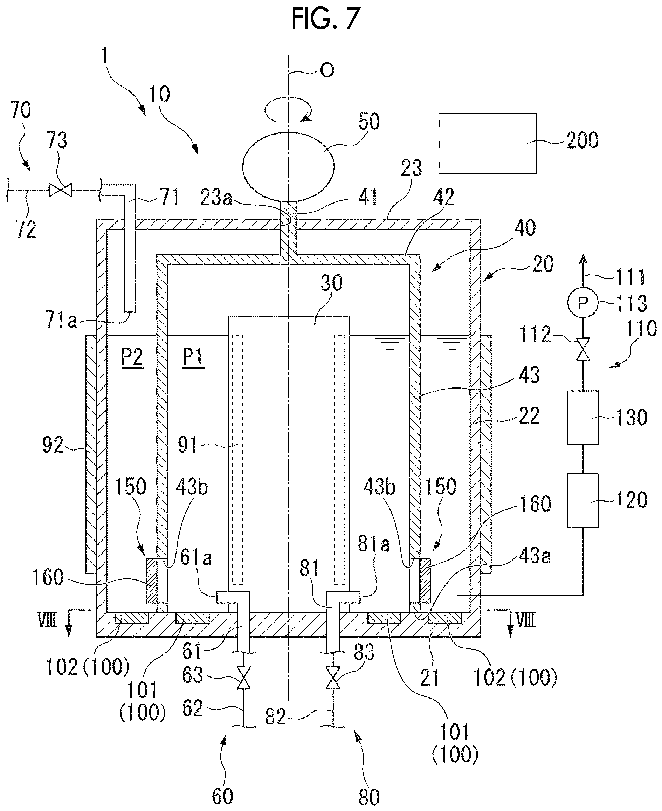

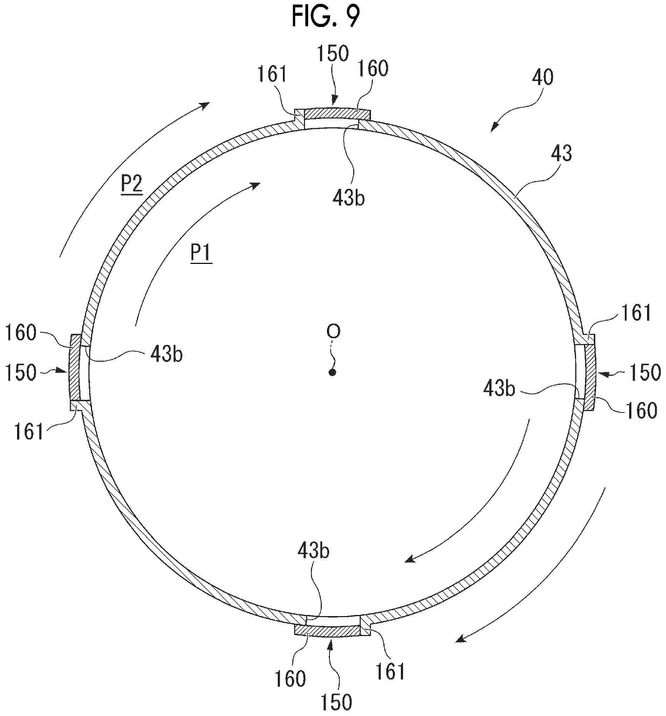

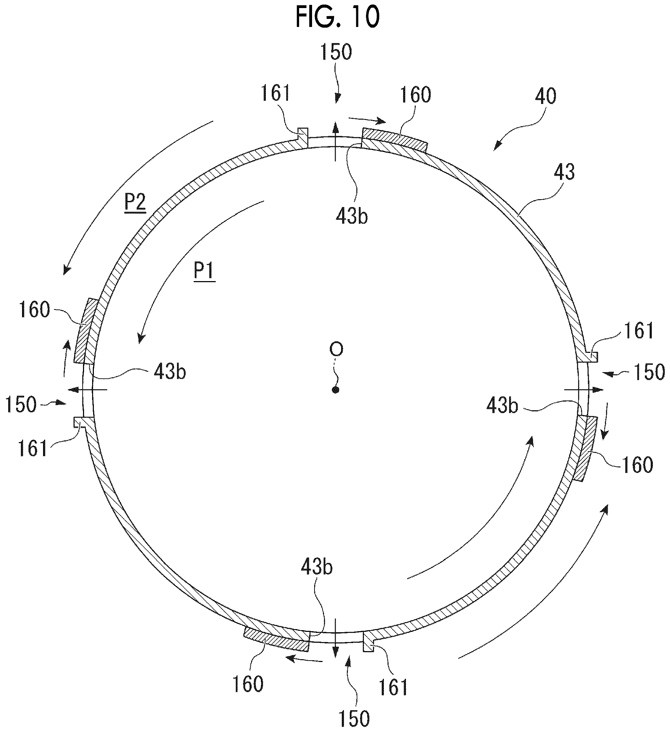

Next, a second embodiment of the present disclosure will be described with reference to FIGs. 7 to 13. In FIGs. 7 to 13, components that are the same as the first embodiment will be assigned with the same reference signs, and detailed description thereof will be omitted.

In the bioreactor system 1 of the second embodiment, the configurations of the drive unit 50, the oxygen supply unit 100, and the transfer mechanism 150 are different from those of the first embodiment.

### <Drive Unit>

The drive unit 50 can rotationally drive the rotating unit 40 in both the forward direction and the reverse direction. That is, the drive unit 50 rotationally drives the rotating cylinder 43 in the forward and reverse directions. It should be noted that, in the culture steps S4 and S6, the culture execution unit 204 controls the drive unit 50 via the rotation control unit 213 to rotationally drive the rotating cylinder 43 in the forward direction.

### <Oxygen Supply Unit>

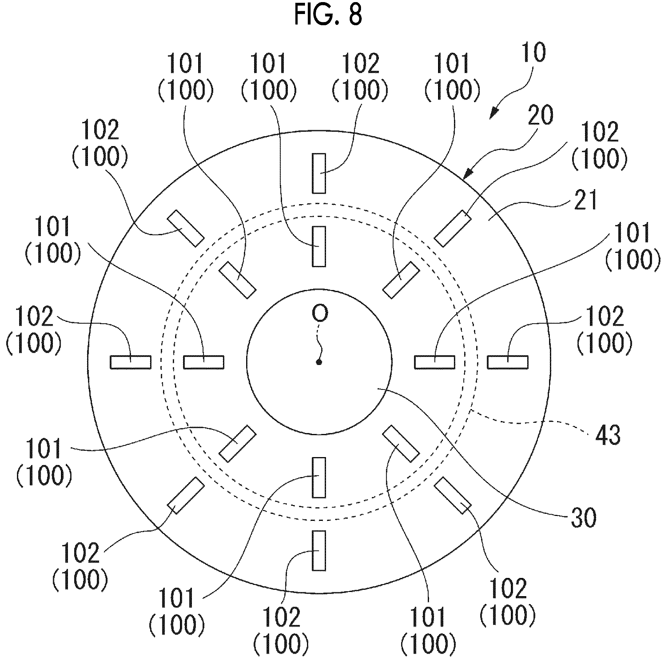

As shown in FIGs. 7 and 8, the oxygen supply unit 100 of the second embodiment includes a first oxygen supply unit 101 and a second oxygen supply unit 102. The first oxygen supply unit 101 and the second oxygen supply unit 102 are spargers that can supply oxygen to a liquid.

The first oxygen supply unit 101 is disposed in a region between the inner cylinder 30 and the rotating cylinder 43 on the upper surface of the lower bottom portion 21 of the casing 20. That is, the first oxygen supply unit 101 is disposed to face only the first pool P1 of the first pool P1 and the second pool P2.

The second oxygen supply unit 102 is disposed in a region between the outer cylinder 22 and the rotating cylinder 43 on the upper surface of the lower bottom portion 21 of the casing 20. That is, the second oxygen supply unit 102 is disposed to face only the second pool P2 of the first pool P1 and the second pool P2.

The first oxygen supply unit 101 and the second oxygen supply unit 102 are independent of each other and release oxygen in respective set supply amounts.

That is, in the culture steps S4 and S6, the oxygen supply control unit 212 controls the first oxygen supply unit 101 and the second oxygen supply unit 102 such that the oxygen supply amount is suitable for the first pool P1 and the second pool P2, which are oxygen supply destinations.

As a result, the first oxygen supply unit 101 supplies oxygen in a supply amount suitable for cell proliferation, for example, to promote cell proliferation in the first pool P1.

In addition, the second oxygen supply unit 102 supplies oxygen in a supply amount suitable for antibody production, for example, to suppress cell proliferation in the second pool P2 and to promote antibody production by the cells.

### <Transfer Mechanism>

The transfer mechanism 150 of the second embodiment is provided in the rotating cylinder 43. As shown in FIGs. 7, 9, and 10, a plurality of opening portions 43b that penetrate the rotating cylinder 43 in the radial direction are provided at the lower portion of the rotating cylinder 43 at a spacing in the circumferential direction.

Furthermore, the transfer mechanism 150 includes a slide door 160 as an opening and closing portion that can open and close the opening portion 43b. The slide door 160 has a plate shape extending in the circumferential direction and the up-down direction, and has a size that can close the opening portion 43b. The slide door 160 is provided to be along the outer peripheral surface of the rotating cylinder 43. The slide door 160 is provided to be movable between a closing position at which the opening portion 43b is closed from the outer peripheral side and an opening position at which the opening portion 43b is opened. The opening position is a position closer to a front side in a rotation direction of the rotating cylinder 43 than the closing position. The slide door 160 is provided to be slidable in the circumferential direction along the outer peripheral surface of the rotating cylinder 43 between the closing position and the opening position.

The transfer mechanism 150 includes a stopper 161. The stopper 161 is provided to protrude from an edge portion of the opening portion 43b of the rotating cylinder 43 on a rear side in the forward rotation direction to the outer peripheral side. The slide door 160 at the closing position is in contact with the stopper 161 from the front side in the forward rotation direction. The transfer of the slide door 160 to the rear side in the forward rotation direction from the closing position is restricted by the stopper 161.

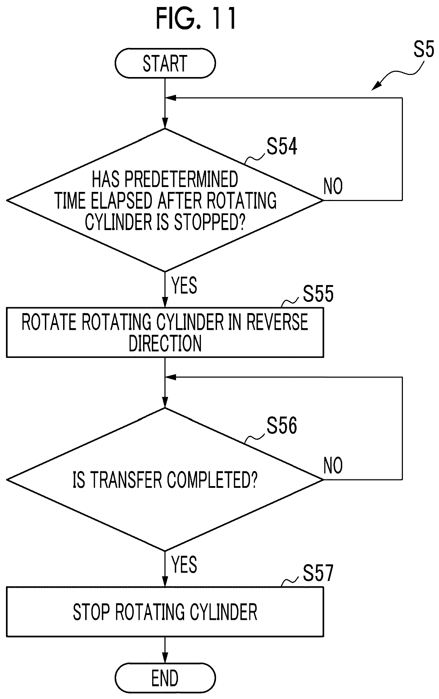

Next, the transfer step S5 of the present embodiment will be described with reference to FIG. 11.

First, the transfer execution unit 205 determines whether or not a predetermined time has elapsed after the first culture step S4 is completed, that is, after the rotation of the rotating cylinder 43 is stopped (step S54). In a case where the predetermined time has not elapsed, the transfer execution unit 205 waits until the predetermined time elapses (step S53: No). In a case where the predetermined time has elapsed, the transfer execution unit 205 proceeds to the next step (step S53: Yes).

As described above, by waiting after the rotation of the rotating cylinder 43 is stopped, the proliferated cells in the culture solution of the first pool P1 precipitate in the lower portion of the first pool P1. As a result, the cell concentration in the lower portion (position in the up-down direction of the opening and closing portion) of the first pool P1 is higher than that in the upper portion.

Next, the transfer execution unit 205 rotationally drives the drive unit 50 in the reverse direction via the rotation control unit 213. As a result, the rotating cylinder 43 is rotationally driven in the reverse direction opposite to the direction in the culture step S4 (step S55).

Here, in a case where the rotating cylinder 43 is rotationally driven in the forward direction, the liquid flow resistance of the first pool P1 and the second pool P2 is applied to the slide door 160, and an external force is applied to the slide door 160 toward the rear side in the forward rotation direction. As a result, the slide door 160 is held in a state of being in contact with the stopper 161 at the closing position. Therefore, in a case where the rotating cylinder 43 is rotationally driven in the forward direction, the slide door 160 is in a state of closing the opening portion 43b, and the liquids of the first pool P1 and the second pool P2 are not mixed through the opening portion 43b.

On the other hand, in a case where the rotating cylinder 43 is rotationally driven in the reverse direction in the transfer step S5, the liquid flow resistance of the first pool P1 and the second pool P2 applies an external force to the slide door 160 toward the rear side in the reverse rotation direction (front side in the forward rotation direction). As a result, the slide door 160 moves relative to the rotating cylinder 43 toward the rear side in the reverse rotation direction. That is, the slide door 160 is slid from the closing position to the opening position. As a result, the first pool P1 and the second pool P2 are in communication with each other in the radial direction through the slide door 160 of the rotating cylinder 43.

Here, the transfer execution unit 205 rotationally drives the rotating cylinder 43 in the reverse direction at a lower rotational speed than in the culture steps S4 and S6 by controlling the rotation control unit 213. As a result, the Taylor vortices are not formed in the first pool P1 and the second pool P2, and active stirring is not performed. On the other hand, the culture solution of the first pool P1 is carried around by the rotating cylinder 43 due to viscosity by the rotating cylinder 43 being in contact with the culture solution of the first pool P1 at a low rotational speed. As a result, a gentle centrifugal force is applied to the culture solution of the first pool P1. The culture solution having a high cell concentration in the first pool P1 is transferred to the second pool P2 through the opening portion 43b by the centrifugal force.

Next, the transfer execution unit 205 determines whether or not the transfer of the culture solution from the first pool P1 to the second pool P2 is completed (step S56). In a case where it is determined that the transfer is not completed (step 56: No), the reverse rotation of the rotating cylinder 43 is continued. In a case where it is determined that the transfer is completed (step 56: Yes), the transfer execution unit 205 stops the reverse rotation of the rotating cylinder 43 by controlling the rotation control unit 213 (step S57). As described above, the transfer step S5 is completed.

It should be noted that the determination of whether or not the transfer by the transfer execution unit 205 is completed may be determined, for example, by whether or not a predetermined time has elapsed from the start of the reverse rotation of the rotating cylinder 43. In addition, the transfer may be determined to be completed in a case where the cell concentration of the liquid of the first pool P1 and the cell concentration of the liquid of the second pool P2 are measured and the cell concentration of the first pool P1 is a predetermined amount or less and the cell concentration of the second pool P2 exceeds the predetermined amount. In addition, the cell concentration of any of the first pool P1 or the second pool P2 may be used as a determination criterion.

### <Operational Effects>

In the bioreactor system 1 of the second embodiment having the above-described configuration, oxygen can be separately supplied to each of the first pool P1 and the second pool P2. Therefore, in a case where cells in different growth phases are present in the first pool P1 and the second pool P2, an oxygen amount suitable for each cell can be supplied, and the culture can be promoted.

In particular, in the present embodiment, by supplying an oxygen amount suitable for cell culture to the first pool P1 and supplying an oxygen amount suitable for antibody production to the second pool P2, two processes of cell culture and antibody production can be efficiently performed.

In addition, according to the transfer mechanism 150 of the present embodiment, the transfer of the culture solution from the first pool P1 to the second pool P2 can be performed only by the reverse rotation of the rotating cylinder 43. Therefore, the transfer of the culture solution can be smoothly and appropriately performed.

Furthermore, the control target is only the drive unit 50 and the rotating cylinder 43, and the transfer valve 152 of the first embodiment is not required. Therefore, the maintainability can be improved by the number of components.

### <Modification Example of Second Embodiment>

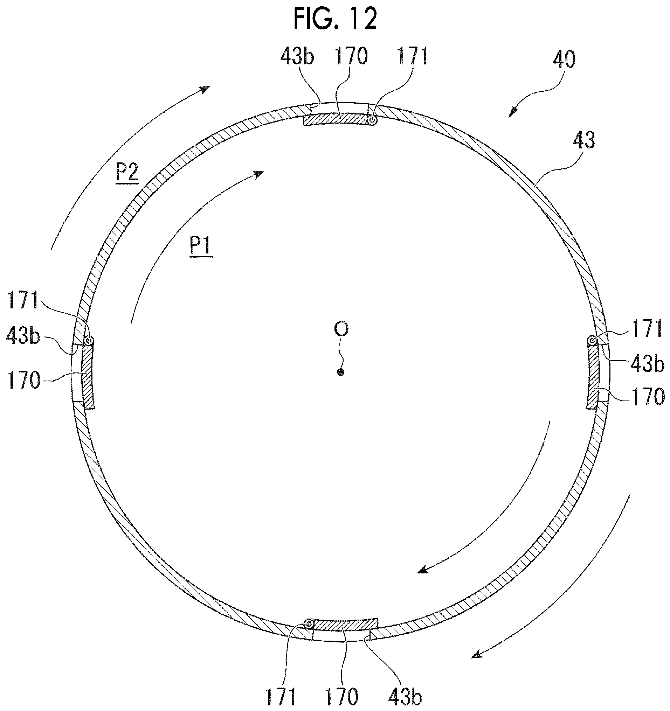

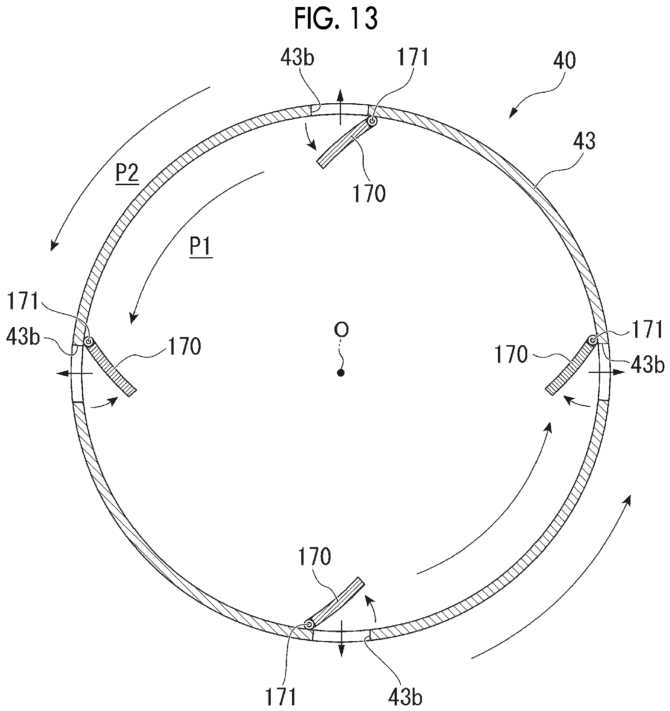

It should be noted that, as a modification example of the second embodiment, the transfer mechanism 150 may have, for example, a configuration shown in FIGs. 12 and 13.

The transfer mechanism 150 of the modification example includes an opening and closing door 170 and a hinge 171 instead of the slide door 160 and the stopper 161 of the second embodiment.

The hinge 171 is provided to extend in the up-down direction on an edge portion of the opening portion 43b on the front side in the forward rotation direction on the inner peripheral surface of the rotating cylinder 43.

The opening and closing door 170 is provided to be rotatable about a rotation axis extending in the up-down direction by the hinge 171 inside the rotating cylinder 43. The opening and closing door 170 is rotated between the closing position and the opening position. As shown in FIG. 12, the closing position is a position at which the opening and closing door 170 is disposed along the shape of the cylindrical surface to close the opening portion 43b. As shown in FIG. 13, the opening position is a position at which the opening and closing door 170 is rotated from the closing position toward the inside of the rotating cylinder 43 to open the opening portion 43b.

In a case where the rotating cylinder 43 is rotationally driven in the forward direction, the liquid flow resistance of the first pool P1 is applied to the opening and closing door 170, and an external force is applied to the opening and closing door 170 toward the rear side in the forward rotation direction, that is, the front side in the reverse rotation direction. As a result, the opening and closing door 170 is held in a state of being closed at the closing position, and the opening portion 43b is closed.

On the other hand, in a case where the rotating cylinder 43 is rotationally driven in the reverse direction, the liquid flow resistance of the first pool P1 applies an external force to the opening and closing door 170 from the closing position toward the opening position. As a result, the opening portion 43b is opened, and the first pool P1 and the second pool P2 are in a communication state.

Therefore, as in the second embodiment, the culture solution can be transferred from the first pool P1 to the second pool P2.

### <Third Embodiment>

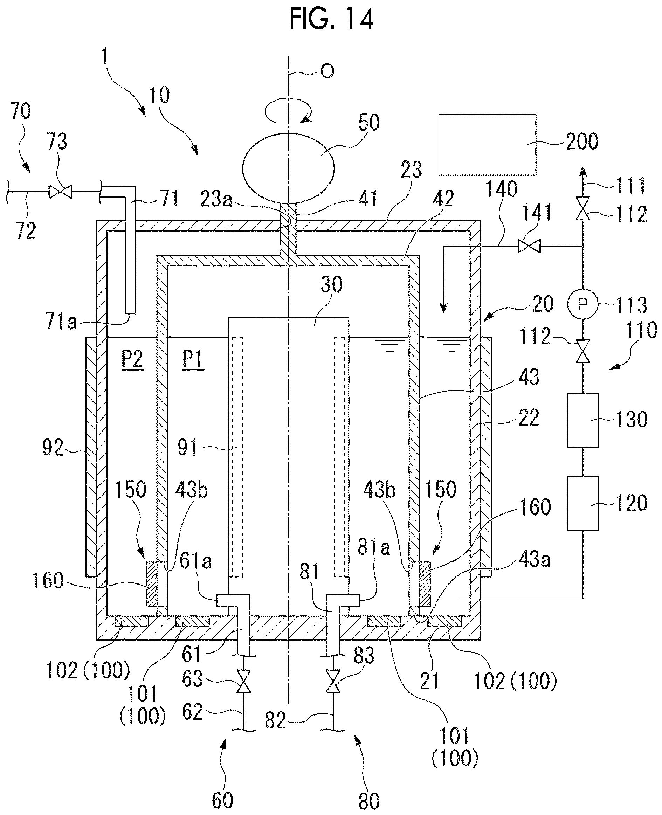

Next, the bioreactor system 1 of the third embodiment will be described with reference to FIGs. 14 to 16. In FIGs. 14 to 16, the same components as those of the second embodiment are designated by the same reference numerals, and detailed description thereof will not be repeated.

In the third embodiment, the removal unit 110 further includes a circulation line 140 and a circulation valve 141.

### <Circulation Line>

One end of the circulation line 140 is connected to a portion between the removal pump 113 and the discharge valve 112 in the discharge line 111. The other end of the circulation line 140 is connected to the second pool P2. The other end of the circulation line 140 is provided to face the culture solution of the second pool P2 from above.

### <Circulating Valve>

The circulation valve 141 is provided on the circulation line 140. The circulation valve 141 switches between a circulation state and a non-circulation state of the circulation line 140 by opening and closing.

### <Control Device>

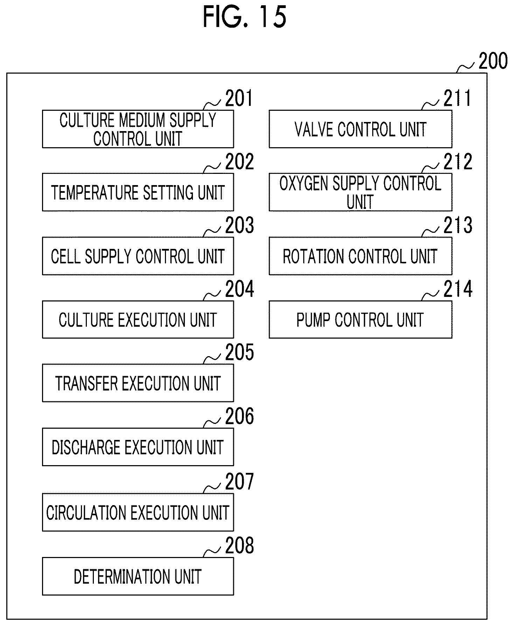

As shown in FIG. 15, the control device 200 of the third embodiment includes a circulation execution unit 207 and a determination unit 208 in addition to the configuration of the control device 200 of the first embodiment shown in FIG. 3.

The circulation execution unit 207 performs opening and closing control of the circulation valve 141 via the valve control unit 211 and controls the removal pump 113 via the pump control unit 214.

The determination unit 208 determines whether or not to perform continuous operation.

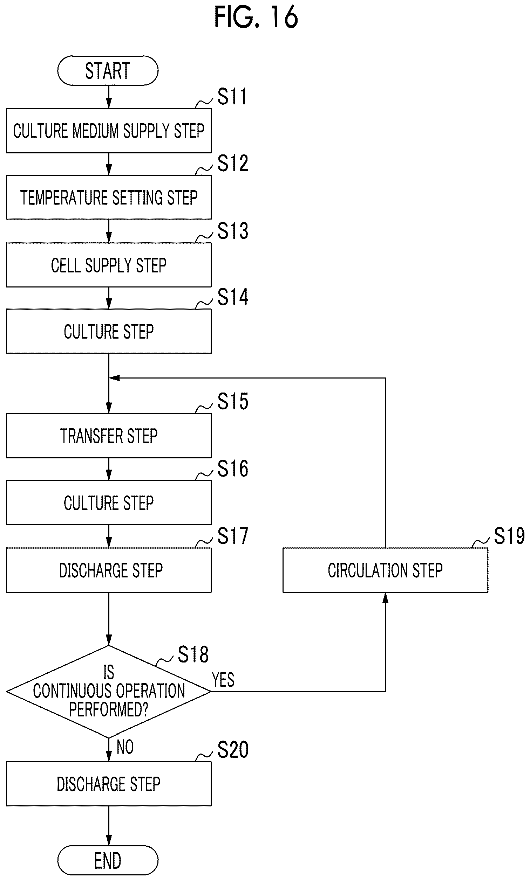

Next, a processing procedure of the control device 200 (operation method of the bioreactor 10) of the third embodiment will be described with reference to the flowchart shown in FIG. 16.

First, as in the first embodiment, a culture medium supply step S11, a temperature setting step S12, a cell supply step S13, a culture step S14, and a transfer step S15 are sequentially executed. It should be noted that, in the configuration of the bioreactor system 1 of the third embodiment shown in FIG. 14, the transfer step S15 is performed by the same method as in the second embodiment, but may be performed by the same method as in the first embodiment.

After the transfer step S15, the same cell supply step S16 as described above is executed again. Here, by executing the transfer step S15, most of the cells in the first pool P1 are transferred to the second pool P2. Therefore, the first pool P1 is in a state where the cells are reduced. By executing the cell supply step S16, the cells are present in both the first pool P1 and the second pool P2.

It should be noted that, in a case where the number of cells in the first pool P1 is secured after the transfer step S15, the cell supply step S16 may be omitted.

Next, the culture execution unit 204 executes the same culture step S17 as described above again. As a result, the Taylor vortices are formed in the first pool P1 and the second pool P2 by the rotation of the rotating cylinder 43. In the present embodiment, since the cells are present in both the first pool P1 and the second pool P2, the cell culture proceeds in both the first pool P1 and the second pool P2. That is, the cell proliferation proceeds in the first pool P1, and the antibody production proceeds in the second pool P2.

After the culture step S17 following the transfer step S15, the determination unit 208 determines whether or not to perform continuous operation (determination step S18).

The determination of whether or not to perform continuous operation is determined, for example, by whether or not the required amount of antibody production is reached. That is, in a case where the discharge step S20 is executed in the next step, in a case where it is not expected that the target amount of antibody production will be reached by acquiring the antibody in the discharge step S20, the determination unit 208 determines to perform the continuous operation. On the other hand, in a case where it is expected that the target amount of antibody production will be reached, the determination unit 208 determines not to perform the continuous operation. It should be noted that the determination unit 208 may determine whether or not to perform the continuous operation based on the number of times the determination is made to perform the continuous operation.

In a case where the determination unit 208 determines to perform the continuous operation (determination step S18: Yes), the circulation execution unit 207 performs a circulation step S19. That is, the circulation execution unit 207 opens the circulation valve 141 via the valve control unit 211 and drives the removal pump 113 via the pump control unit 214. It should be noted that, in this state, the discharge valve 112 is in the closed state.

As a result, the culture solution of the second pool P2 is extracted to the outside via the discharge line 111, and the cells and the antibody are extracted by the cell acquisition unit 120 and the antibody acquisition unit 130. Then, the culture medium remaining after the cells and the antibody are extracted from the culture solution is returned to the second pool P2 via the circulation line 140.

After the circulation step S19, the transfer step S15, the cell supply step S16, the culture step S17, and the determination step S18 are executed again. Here, the cell supply step S16 may be omitted.

In a case where the determination unit 208 determines not to perform the continuous operation (determination step S18: No), the discharge step S20 as in the first embodiment is performed. As a result, the final extraction of the cells and the antibody is performed, and the processing by the control device 200 is completed.

As described above, according to the present embodiment, the cell culture can be performed simultaneously in each of the first pool P1 and the second pool P2. That is, by partitioning the first pool P1 and the second pool P2, the cell culture in different growth phases such as cell proliferation and antibody production can be performed simultaneously. Therefore, the efficiency of the cell culture can be improved, and since the different cell cultures can be performed by only one bioreactor 10, the entire cell culture facility can be made compact.

In addition, by performing the continuous operation, the cell proliferation in different growth phases such as the cell proliferation and the antibody production can be continuously performed simultaneously, so that the amount of antibody productions can be increased. Furthermore, in the circulation step, by returning the culture medium to the second pool P2, the reuse of the resources necessary for the cell culture can be achieved.

It should be noted that, in a case of performing the continuous operation as in the present embodiment, the first culture medium and the second culture medium may be appropriately supplied to the first pool P1 and the second pool P2 during the operation. In addition, only the nutrients in the first culture medium and the second culture medium may be appropriately supplied. As a result, the nutrients necessary for the cells can be maintained, and the continuous operation can be performed without reducing the speed of the culture.

### <Fourth Embodiment>

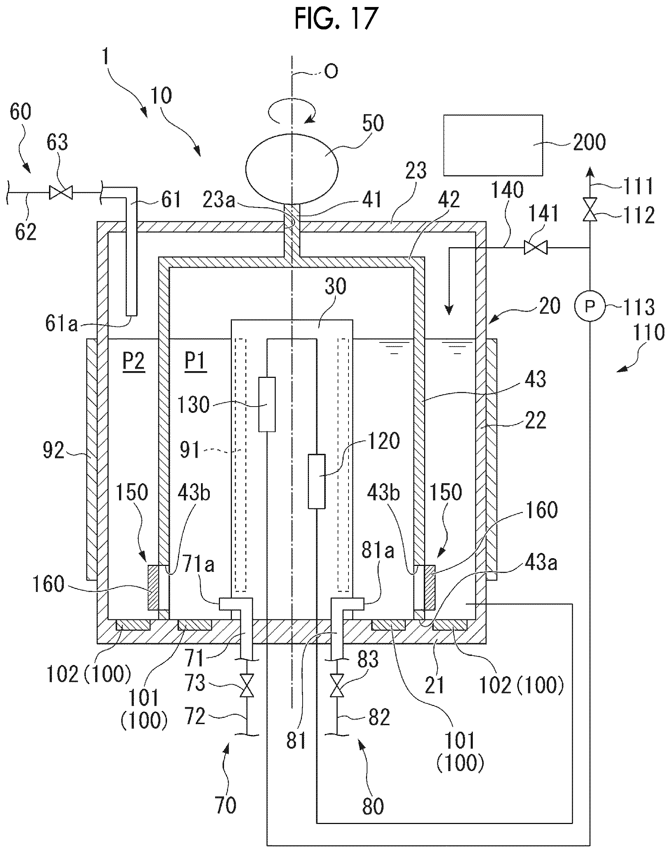

Next, the bioreactor system 1 of the fourth embodiment will be described with reference to FIG. 17. In FIG. 17, components that are the same as the first embodiment will be assigned with the same reference signs, and detailed description thereof will be omitted.

In the third embodiment, a part of the discharge line 111 passes through the inside of the inner cylinder 30. That is, the discharge line 111 is disposed to penetrate the lower bottom portion 21 from below to above, and reaches the inside of the inner cylinder 30. Then, the discharge line 111 extends upward to the upper side of the inner cylinder 30, is folded downward, and penetrates the lower bottom portion 21 from above to below to reach the outside of the inner cylinder 30.

The cell acquisition unit 120 and the antibody acquisition unit 130 on the discharge line 111 are provided inside the inner cylinder 30. It should be noted that only any one of the cell acquisition unit 120 or the antibody acquisition unit 130 may be provided inside the inner cylinder 30. That is, at least one of the cell acquisition unit 120 or the antibody acquisition unit 130 may be provided inside the inner cylinder 30.

In addition, the removal pump 113 and the discharge valve 112 are provided outside the inner cylinder 30, but may be provided inside the inner cylinder 30. Furthermore, a part of the circulation line 140 and the circulation valve 141 may be provided inside the inner cylinder 30.

As a result, the effective use of the space inside the inner cylinder 30 can be achieved. In another embodiment, by providing the configuration provided outside the casing 20 inside the inner cylinder 30, the entire device can be made compact. Furthermore, by aggregating and disposing various configurations inside the inner cylinder 30, the maintainability can be improved.

### <Fifth Embodiment>

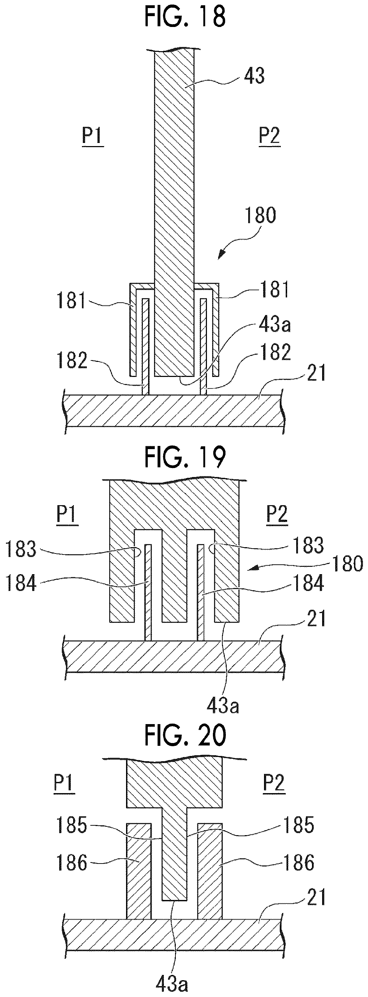

Next, the bioreactor system 1 of the fifth embodiment will be described with reference to FIG. 18. In FIG. 18, components that are the same as the first embodiment will be assigned with the same reference signs, and detailed description thereof will be omitted.

### <Seal Portion>

In the fifth embodiment, a seal portion 180 that seals between the lower end edge portion 43a of the rotating cylinder 43 and the upper surface of the lower bottom portion 21 over the circumferential direction is provided.

The seal portion 180 includes a rotating seal piece 181 and a fixed seal piece 182.

The rotating seal piece 181 is provided over the entire periphery on each of the inner peripheral surface and the outer peripheral surface of the rotating cylinder 43. The rotating seal piece 181 has a shape that protrudes from the inner peripheral surface and the outer peripheral surface of the rotating cylinder 43 and then extends downward.

A pair of the fixed seal pieces 182 is provided on the upper surface of the lower bottom portion 21. The fixed seal piece 182 is provided to correspond to each of the pair of rotating seal pieces 181 on the upper surface of the lower bottom portion 21. The fixed seal piece 182 protrudes upward from the lower bottom portion 21 and is provided to enter between the rotating cylinder 43 and the rotating seal piece 181 over the entire periphery. With the rotating seal piece 181 and the fixed seal piece 182, a labyrinth mechanism is configured in the radial direction of the clearance.

In the present embodiment, by providing the seal portion 180, the transfer of the cells and the culture medium through the clearance between the rotating cylinder 43 and the lower bottom portion 21 can be suppressed. As a result, the first pool P1 and the second pool P2 can be appropriately partitioned, so that the cell culture corresponding to the growth phase can be reliably performed in each pool.

### <First Modification Example of Fifth Embodiment>

It should be noted that, as a first modification example of the fifth embodiment, the seal portion 180 may have, for example, a configuration shown in FIG. 19. The seal portion 180 of the first modification example is configured by an annular groove 183 formed in the rotating cylinder 43 and fins 184 and 186 provided on the lower bottom portion 21.

The annular groove 183 is recessed from the lower end edge portion 43a of the rotating cylinder 43 toward the upper side and extends in an annular shape over the entire periphery. A plurality (two in the present embodiment) of the annular grooves 183 are formed at a spacing in the radial direction.

A plurality (two in the present embodiment) of the fins 184 and 186 are provided to correspond to the annular groove 183. The lower end of each of the fins 184 and 186 is fixed to the upper surface of the lower bottom portion 21 and extends upward to enter each annular groove 183.

As a result, the labyrinth mechanism can be configured in the clearance. In addition, since there is no protrusion from the inner surface and the outer surface of the rotating cylinder 43 as compared with the fourth embodiment, the occurrence of turbulence in the first pool P1 and the second pool P2 is suppressed. Therefore, appropriate Taylor vortices can be formed in the first pool P1 and the second pool P2.

### <Second Modification Example of Fifth Embodiment>

In addition, as a second modification example of the fifth embodiment, the seal portion 180 may have, for example, a configuration shown in FIG. 20. The seal portion 180 of the second modification example is configured by a notch portion 185 formed in the rotating cylinder 43 and fins 184 and 186 provided on the lower bottom portion 21.

A pair of the notch portions 185 is provided to be recessed from the outer peripheral surface and the inner peripheral surface of the rotating cylinder 43 in the lower portion of the rotating cylinder 43 and to be open downward. As a result, the lower end of the rotating cylinder 43 is formed to have a smaller radial thickness than the upper portion.

The fins 184 and 186 have an annular shape that protrudes from the upper surface of the lower bottom portion 21 and extends over the entire circumferential direction, as in the first modification example. The fins 184 and 186 are provided to be accommodated in each notch portion 185 to correspond to the pair of notch portions 185. The inner peripheral surface of the rotating cylinder 43 and the inner peripheral surfaces of the fins 184 and 186 on the radial inner side of the pair of fins 184 and 186 are positioned in the same plane on the same cylindrical surface. The outer peripheral surface of the rotating cylinder 43 and the outer peripheral surfaces of the fins 184 and 186 on the radial outer side of the pair of fins 184 and 186 are positioned in the same plane on the same cylindrical surface.

As a result, as in the first modification example, the labyrinth mechanism can be configured, and since the protrusion is not formed in the first pool P1 and the second pool P2, appropriate Taylor vortices can be formed in the first pool P1 and the second pool P2.

### <Sixth Embodiment>

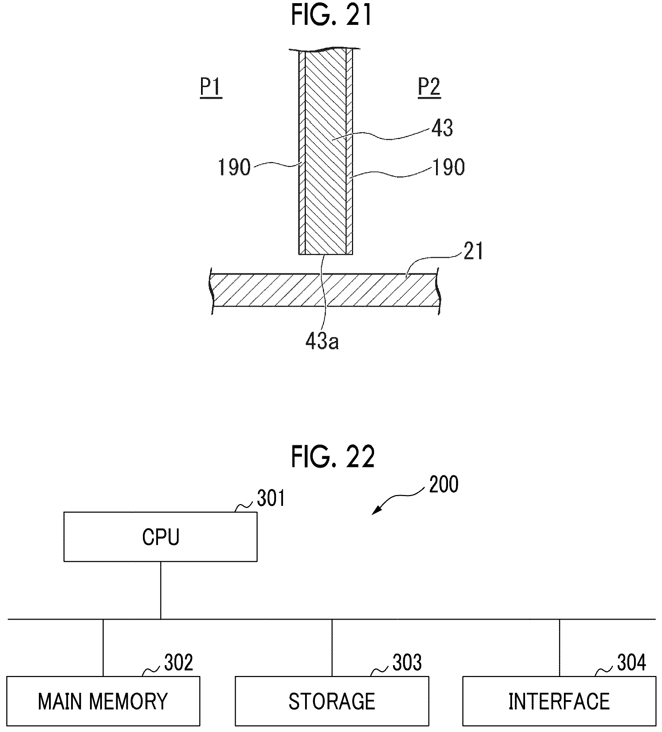

Next, the bioreactor system 1 of the sixth embodiment will be described with reference to FIG. 21. In FIG. 21, components that are the same as the first embodiment will be assigned with the same reference signs, and detailed description thereof will be omitted.

### <Low Thermal Conductivity Member>

In the sixth embodiment, a low thermal conductivity member 190 is provided on the surface of the rotating cylinder 43. The low thermal conductivity member 190 is provided along the inner peripheral surface and the outer peripheral surface of the rotating cylinder 43. The low thermal conductivity member 190 is provided over an entire region of at least a portion of the inner peripheral surface and the outer peripheral surface of the rotating cylinder 43 that is in contact with the culture solution. The low thermal conductivity member 190 may be provided on at least one of the inner peripheral surface and the outer peripheral surface of the rotating cylinder 43.

The low thermal conductivity member 190 is made of a material having a lower thermal conductivity than a material constituting the rotating cylinder 43. For example, in a case where the low thermal conductivity member 190 is made of a steel material such as stainless steel, a ceramic applied by thermal spraying, rubber attached separately, or the like can be used as the material of the low thermal conductivity member 190.

The presence of the low thermal conductivity member 190 can suppress the transfer of heat between the first pool P1 and the second pool P2. Therefore, the temperature control of the first pool P1 and the second pool P2 by the first temperature adjusting unit 91 and the second temperature adjusting unit can be more appropriately performed. Therefore, the cell culture corresponding to the growth phase in the first pool P1 and the second pool P2 can be more appropriately performed.

It should be noted that, in order to suppress the transfer of heat between the first pool P1 and the second pool P2, for example, the material itself constituting the rotating cylinder 43 may be made of a material having a lower thermal conductivity than the outer cylinder 22. As a result, the transfer of heat between the first pool P1 and the second pool P2 can be suppressed while the temperature in the second pool P2 is appropriately adjusted from the outside of the outer cylinder 22 by the second temperature adjusting unit 92, and the temperature can be set independently in each pool.

### <Other Embodiments>

Although the embodiments of the present invention have been described hereinbefore, the present invention is not limited thereto and can undergo some changes as appropriate without departing from the technical spirit of the invention.

For example, the operation method of the bioreactor 10 according to the embodiment has been described as a configuration in which the control device 200 performs each step, but all or a part of the steps may be performed by an operator.

For example, the culture medium supply step, the cell supply step, the transfer step, the circulation step, and the discharge step may be performed manually by the operator.

In addition, the temperature setting unit 202 may receive an input from the operator and control the first temperature adjusting unit and the second temperature adjusting unit in accordance with the input.

Furthermore, the rotation control unit 213 may receive an input from the operator and rotate the rotating cylinder 43 via the drive unit 50 in accordance with the input.

Similarly, the oxygen supply unit 100 may receive an input from the operator and control the oxygen supply unit 100 in accordance with the input.

In the embodiment, the cells proliferated in the first pool P1 are transferred to the second pool P2 and used, but the simultaneous culture may be performed by supplying the cells in different growth phases to the first pool P1 and the second pool P2 from the outside, respectively.

### <Hardware Configuration>

The process of the processing by the control device 200 described above is stored in the form of a program in a recording medium that can be read by a computer, and the computer reads out and executes this program, so that the processing is performed. A specific example of the computer will be described below.

As shown in FIG. 22, the computer includes a CPU 301, a main memory 302, a storage 303, and an interface 304.

For example, the above-described computing device is mounted on a computer. Then, the operation of each processing unit described above is stored in the storage 303 in the form of a program. The CPU 301 reads the program from the storage 303, develops the program in the main memory 302, and executes the above processing according to the program. In addition, the CPU 301 secures a storage area in the main memory 302 according to the program.

As examples of the storage 303, a hard disk drive (HDD), a solid-state drive (SSD), a magnetic disk, a magneto-optical disk, a compact disc read-only memory (CD-ROM), a digital versatile disc read-only memory (DVD-ROM), a semiconductor memory, and the like can be given. The storage 303 may be an internal medium directly connected to a bus of the computer or may be an external medium that is connected to the computer via the interface 304 or a communication line. Further, in a case where this program is distributed to the computer via a communication line, the computer receiving the distribution may develop the program in the main memory 302 and execute the above processing. The storage 303 is a non-temporary tangible storage medium.

In addition, the program may realize some of the functions described above. Further, the program may be a file that can realize the functions described above in combination with a program already recorded in the computer, that is, a so-called differential file (differential program).

A custom large scale integrated circuit (LSI) such as a programmable logic device (PLD), an application specific integrated circuit (ASIC), a graphics processing unit (GPU), and a processing device similar thereto may be provided in addition to the above-described configuration or instead of the above-described configuration. As examples of the PLD, a programmable array logic (PAL), a generic array logic (GAL), a complex programmable logic device (CPLD), and a field programmable gate array (FPGA) can be given. In this case, a part or all of the functions that are realized by the processor may be realized by the integrated circuit.

### <Supplementary Notes>

The embodiments described above can be understood as follows.

(1) A bioreactor 10 according to a first aspect includes an outer cylinder 22 centered on an axis line O extending in a vertical direction; an inner cylinder 30 provided inside the outer cylinder 22 around the axis line O; a rotating cylinder 43 disposed between the outer cylinder 22 and the inner cylinder 30 to partition a first pool P1 between the rotating cylinder 43 and the inner cylinder 30, and a second pool P2 between the rotating cylinder 43 and the outer cylinder 22 and rotatable about the axis line O; a drive unit 50 configured to rotationally drive the rotating cylinder 43 about the axis line O; a first supply port 61a configured to supply liquid to the first pool P1; and a second supply port 71a configured to supply liquid to the second pool P2.

As a result, different culture media can be supplied to the first pool P1 and the second pool P2. In addition, by rotating the rotating cylinder 43, the Taylor vortices can be formed in both the first pool P1 and the second pool P2. Therefore, the culture of cells in different growth phases can be simultaneously performed in a compact configuration.

(2) A bioreactor 10 according to a second aspect is the bioreactor 10 of (1), further including a first culture medium supply unit 60 configured to supply, through the first supply port 61a, a first culture medium as the liquid to the first pool P1; and a second culture medium supply unit 70 configured to supply, through the second supply port 71a, a second culture medium having a component different from the first culture medium as the liquid to the second pool P2.

As a result, the growth environment of the cells depending on the different growth phases can be constructed in each of the first pool P1 and the second pool P2.

(3) A bioreactor 10 according to a third aspect is the bioreactor 10 of (1) or (2), further including a first temperature adjusting unit 91 configured to adjust a temperature of the first pool P1; and a second temperature adjusting unit 92 configured to adjust a temperature of the second pool P2 independently of the first temperature adjusting unit 91.

As a result, the first pool P1 and the second pool P2 can be set to an appropriate temperature depending on the growth phase of the cells.

(4) The bioreactor 10 according to a fourth aspect is the bioreactor 10 of (3), further including a low thermal conductivity member 190 that is provided on a surface of the rotating cylinder 43 and has a lower thermal conductivity than the rotating cylinder 43.

As a result, heat transfer between the first pool P1 and the second pool P2 can be suppressed.

(5) A bioreactor 10 according to a fifth aspect is the bioreactor 10 according to (3) or (4), in which the rotating cylinder 43 is made of a material having a lower thermal conductivity than the outer cylinder 22.

As a result, heat transfer between the first pool P1 and the second pool P2 can be suppressed.

(6) The bioreactor 10 according to a sixth aspect is the bioreactor 10 of any one of (1) to (5), further including an oxygen supply unit 100 configured to supply oxygen to the first pool P1 and the second pool P2.

As a result, oxygen necessary for the cell culture can be supplied to the first pool P1 and the second pool P2.

(7) A bioreactor 10 according to a seventh aspect is the bioreactor 10 of (6), in which the oxygen supply unit 100 includes a first oxygen supply unit 101 configured to supply oxygen to the first pool P1, and a second oxygen supply unit 102 configured to supply oxygen to the second pool P2 independently of the first oxygen supply unit 101.

As a result, the appropriate oxygen supply amount can be set for each of the first pool P1 and the second pool P2 depending on the growth phase of the cells.

(8) The bioreactor 10 according to an eighth aspect is the bioreactors 10 of any one of (1) to (7), further including a cell supply unit 80 configured to supply cells to the first pool P1; and a transfer mechanism 150 configured to transfer at least a part of the liquid in the first pool P1 to the second pool P2.

As a result, for example, after the cells are proliferated in the first pool P1, in a case where the cells progress to the next growth phase, the cells can be transferred to the second pool P2 to perform the culture suitable for the growth phase.

(9) A bioreactor 10 according to a ninth aspect is the bioreactor 10 of (8), in which the transfer mechanism 150 includes a transfer tube connecting the first pool P1 and the second pool P2, and a transfer valve 152 provided in the transfer tube and configured to be opened and closed.

As a result, the culture medium and the cells can be transferred from the first pool P1 to the second pool P2.

(10) A bioreactor 10 according to a tenth aspect is the bioreactor 10 of (8), in which the drive unit 50 is configured to rotationally drive the rotating cylinder 43 in forward and reverse directions, the rotating cylinder 43 has an opening portion 43b that penetrates the rotating cylinder 43 in a radial direction, and the transfer mechanism 150 includes an opening and closing portion configured to close the opening portion 43b when the rotating cylinder 43 rotates in the forward direction and to open the opening portion 43b when the rotating cylinder 43 rotates in the reverse direction.

As a result, the culture medium and the cells can be easily transferred from the first pool P1 to the second pool P2.

(11) A bioreactor 10 according to an eleventh aspect is the bioreactor 10 of (10), in which the transfer mechanism 150 includes a slide door 160 that is slidable between a closing position in which the opening portion 43b is closed, and an opening position located on a forward side of the closing position in a forward rotation direction, in which the opening portion 43b is opened.

As a result, the culture medium and the cells can be easily transferred from the first pool P1 to the second pool P2.

(12) A bioreactor 10 according to a twelfth aspect is the bioreactor 10 of (10), in which the transfer mechanism 150 includes an opening and closing door 170 provided on a forward side in the forward rotation direction of the opening portion 43b to be rotatable about a rotation axis line extending in a vertical direction, and the opening and closing door 170 is rotatable between a closing position in which the opening portion 43b is closed, and an opening position that is separated from the opening portion 43b to open the opening portion 43b.

As a result, the culture medium and the cells can be easily transferred from the first pool P1 to the second pool P2.

(13) A bioreactor 10 according to a thirteenth aspect is the bioreactor 10 of any one of (1) to (12), further including
a removal unit 110 including a discharge line 111 configured to allow liquid in the second pool P2 to be removed to the outside, a cell acquisition unit 120 provided in the discharge line 111 and extracting cells from the liquid, and an antibody acquisition unit 130 provided in the discharge line 111 and extracting an antibody produced by the cells from the liquid.

As a result, the liquid can be extracted from the second pool P2, and the cells and the antibody can be extracted from the liquid.

(14) A bioreactor 10 according to a fourteenth aspect is the bioreactor 10 of (13), in which the discharge line 111 passes through an inside of the inner cylinder 30, and at least one of the cell acquisition unit 120 and the antibody acquisition unit 130 is provided inside the inner cylinder 30.

As a result, the space inside the inner cylinder 30 can be effectively used, and the installation space of the cell acquisition unit 120 and the antibody acquisition unit 130 can be efficiently used.

(15) A bioreactor 10 according to a fifteenth aspect is the bioreactor 10 of (13) or (14), in which the removal unit 110 includes a circulation line 140 connected on a downstream side of the cell acquisition unit 120 and the antibody acquisition unit 130 in the discharge line, the circulation line 140 being configured to return the liquid to the second pool P2.

As a result, the reuse of the culture medium and the cells can be achieved. In addition, since the continuous culture can be performed, the efficiency of the antibody production can be improved.

(16) A bioreactor 10 according to a sixteenth aspect is the bioreactor 10 of any one of (1) to (15), further including a lower bottom portion 21 closing the outer cylinder 22 from below, in which a lower end edge portion 43a of the rotating cylinder 43 faces a lower end of the lower bottom portion 21 through a clearance.

As a result, the rotating cylinder 43 can be smoothly rotated while the liquid of the first pool P1 and the liquid of the second pool P2 are prevented from being unintentionally mixed.

(17) A bioreactor 10 according to a seventeenth aspect is the bioreactor 10 of (16), further including a seal portion 180 sealing between the lower end edge portion 43a of the rotating cylinder 43 and the upper surface of the lower bottom portion 21.

As a result, the mixing of the liquid of the first pool P1 and the liquid of the second pool P2 can be further suppressed.

(18) A bioreactor system 1 according to an eighteenth includes the bioreactor 10 according to (1); and a control device 200 that controls the bioreactor 10, wherein the bioreactor 10 includes a first culture medium supply unit 60 configured to supply, through the first supply port 61a, a first culture medium as the liquid to the first pool P1, and a second culture medium supply unit 70 configured to supply, through the second supply port 71a, a second culture medium having a component different from the first culture medium as the liquid to the second pool P2 and the control device 200 includes a culture medium supply control unit 201 performing a culture medium supply step in which the first culture medium is supplied to the first pool P1 by controlling the first culture medium supply unit 60, and the second culture medium is supplied to the second pool P2 by controlling the second culture medium supply unit 70.

As a result, the growth environment of the cells in different growth phases can be constructed in a compact configuration.

(19) A bioreactor system 1 according to a nineteenth aspect is the bioreactor system 1 of (18), in which the bioreactor 10 includes the cell supply unit 80 that supplies cells to the first culture medium of the first pool P1, and the control device 200 includes a cell supply control unit 203 that executes a cell supply step of supplying the cells to the first pool P1 by controlling the cell supply unit 80 after the culture medium supply step, and a culture execution unit 204 that executes a culture step of rotationally driving the rotating cylinder 43 by controlling the drive unit 50 after the cell supply step.

As a result, the cell proliferation can be performed in the first pool P1.

(20) A bioreactor system 1 according to a twentieth aspect is the bioreactor system 1 of (19), in which the bioreactor 10 includes the transfer mechanism 150 that moves at least a part of the liquid of the first pool P1 to the second pool P2, the control device 200 includes a transfer execution unit 205 that executes a transfer step of moving the liquid of the first pool P1 to the second pool P2 by controlling the transfer mechanism 150 after the culture step, and the culture execution unit 204 executes the culture step again after the transfer step.

By moving the cells proliferated in the first pool P1 to the second pool P2, the culture corresponding to the growth phase of the cells can be sequentially performed in one device. For example, in the second phase, the antibody production can be efficiently performed by setting the environment suitable for the cells to produce the antibody.

(21) A bioreactor system 1 according to a twenty-first aspect is the bioreactor system 1 of (20), in which the transfer mechanism 150 includes a transfer pipe that connects the first pool P1 and the second pool P2, and a transfer valve 152 that is provided in the transfer pipe and configured to be openable and closable, and the transfer execution unit 205 moves the liquid of the first pool P1 to the second pool P2 by controlling the transfer valve 152 as the transfer step.

As a result, the transfer of the cells from the first pool P1 to the second pool P2 can be easily performed.

(22) A bioreactor system 1 according to a twenty-second aspect is the bioreactor system 1 of (20), in which the drive unit 50 is capable of driving the rotating cylinder 43 in the forward and reverse directions, the culture execution unit 204 that rotationally drives the rotating cylinder 43 in the forward direction by controlling the drive unit 50, the rotating cylinder 43 that has the opening portion 43b that penetrates the rotating cylinder 43 in the radial direction, the transfer mechanism 150 that includes an opening and closing portion that closes the opening portion 43b in a case where the rotating cylinder 43 is rotationally driven in the forward direction and that opens the opening portion 43b in a case where the rotating cylinder 43 is rotationally driven in the reverse direction, and the transfer execution unit 205 that rotationally drives the rotating cylinder 43 in the reverse direction at a rotational speed lower than the forward rotational driving by controlling the drive unit 50 as the transfer step.

As a result, the cells and the culture medium of the first pool P1 can be transferred to the second pool P2 by only the rotational driving of the rotating cylinder 43. That is, since the transfer of the cells can be performed by setting only the rotating cylinder 43 as the control target, a simple configuration can be adopted.

(23) A bioreactor system 1 according to a twenty-third aspect is the bioreactor system 1 of any one of (20) to (22), in which the bioreactor 10 includes the removal unit 110 that includes the discharge line 111 that can discharge the liquid of the second pool P2 to the outside, the cell acquisition unit 120 that is provided in the discharge line 111 and extracts the cells from the liquid, and the antibody acquisition unit 130 that is provided in the discharge line 111 and extracts the antibody produced by the cells from the liquid, and the control device 200 includes a discharge execution unit 206 that executes a discharge step of extracting and discharging the liquid from the second pool P2 by controlling the removal unit 110 after the culture step after the transfer step is completed.

As a result, the liquid of the second pool P2 after the antibody production can be extracted to the outside, and the cells and the antibody can be extracted from the liquid.

(24) A bioreactor system 1 according to a twenty-fourth aspect is the bioreactor system 1 of (20), in which the removal unit 110 includes a circulation line 140 that is connected to a downstream side of the cell acquisition unit 120 and the antibody acquisition unit 130 in the discharge line 111 and that can return the liquid to the second pool P2, the cell supply control unit 203 that executes the cell supply step again between the transfer step and the culture step after the transfer step, the control device 200 that further includes a determination unit 208 that determines whether or not to perform continuous operation between the culture step after the transfer step and the discharge step, a discharge execution unit 206 that executes the discharge step in a case where the determination unit 208 determines not to perform the continuous operation and that executes a circulation step of returning the liquid extracted from the second pool P2 to the second pool P2 via the circulation line 140 by controlling the removal unit 110 in a case where the determination unit 208 determines to perform the continuous operation, and the transfer execution unit 205 that executes the transfer step again after the circulation step.

As a result, the reuse of the culture medium can be achieved. In addition, by repeating the circulation step, the cell proliferation in the first pool P1 and the antibody production in the second pool P2 can be sequentially performed. That is, since the continuous culture can be performed, the production efficiency of the antibody can be improved.

(25) A bioreactor system 1 according to a twenty-fifth aspect includes the bioreactor 10 according to (1), and a control device 200 that controls the bioreactor 10, in which the bioreactor 10 includes the first temperature adjusting unit 91 that adjusts a temperature of the first pool P1, and the second temperature adjusting unit 92 that adjusts a temperature of the second pool P2,
the control device 200 includes a temperature setting unit 202 that controls the first temperature adjusting unit 91 and the second temperature adjusting unit 92 such that the first pool P1 and the second pool P2 have different temperatures.

As a result, the first pool P1 and the second pool P2 can be set to temperatures suitable for each of the first pool P1 and the second pool P2.

(26) A bioreactor system 1 according to a twenty-sixth aspect includes the bioreactor 10 according to (1) and a control device 200 that controls the bioreactor 10, in which the bioreactor 10 includes a first oxygen supply unit 101 that can supply oxygen to the first pool P1 and a second oxygen supply unit 102 that can supply oxygen to the second pool P2, and the control device 200 includes an oxygen supply control unit 212 that controls the first oxygen supply unit 101 and the second oxygen supply unit 102 such that oxygen supply amounts to the first pool P1 and the second pool P2 are different from each other.

As a result, the oxygen concentrations of the first pool P1 and the second pool P2 can be set to values suitable for each of the first pool P1 and the second pool P2.

(27) A method for operating a bioreactor 10 according to a twenty-seventh aspect includes an outer cylinder 22 centered on an axis line O extending in a vertical direction, an inner cylinder 30 provided inside the outer cylinder 22 around the axis line O, a rotating cylinder 43 disposed between the outer cylinder 22 and the inner cylinder 30 to partition a first pool P1 between the rotating cylinder 43 and the inner cylinder 30, and a second pool P2 between the rotating cylinder 43 and the outer cylinder 22 and rotatable about the axis line O, and a drive unit 50 configured to rotationally drive the rotating cylinder 43 about the axis line O, the method including: a culture medium supply step of supplying a first culture medium to the first pool P1 and supplying a second culture medium having a component different from the first culture medium to the second pool P2.

As a result, the culture corresponding to the growth phase of the cells can be performed in a compact configuration.

(28) A method for operating a bioreactor 10 according to a twenty-eighth aspect is the method for operating a bioreactor 10 of (27), in which a cell supply step of supplying cells to the first pool P1 after the culture medium supply step; and a culture step of rotationally driving the rotating cylinder 43 via the drive unit 50 after the cell supply step.

As a result, the cells can be proliferated in the first pool P1.

(29) A method for operating a bioreactor 10 according to a twenty-ninth aspect is the method for operating a bioreactor 10 of (28), further including: a transfer step of transferring liquid in the first pool P1 to the second pool P2 after the culture step, in which the culture step is performed again after the transfer step.

By moving the cells proliferated in the first pool P1 to the second pool P2, the culture corresponding to the growth phase of the cells can be performed. For example, in the second phase, the antibody production can be efficiently performed by setting the environment suitable for the cells to produce the antibody.

(30) A method for operating a bioreactor 10 according to a thirtieth aspect is the method for operating a bioreactor 10 of (29), further including: a discharge step of extracting the liquid from the second pool P2 and extracting the cells and the antibody produced by the cells from the liquid after the culture step after the transfer step is completed.

As a result, the liquid of the second pool P2 after the antibody production can be extracted to the outside, and the cells and the antibody can be extracted from the liquid.

(31) A method for operating a bioreactor 10 according to a thirty-first aspect is the method for operating a bioreactor 10 of (30), further including: a determination step of determining whether or not to perform continuous operation between the culture step after the transfer step and the discharge step, in which the discharge step is executed in a case where it is determined in the determination step not to perform the continuous operation, and a circulation step of extracting the liquid from the second pool P2, extracting the cells and the antibody from the liquid, and returning the liquid to the second pool P2 after the extraction in a case where it is determined in the determination step to perform the continuous operation, is further included, and the transfer step is executed again after the circulation step.

As a result, the reuse of the culture medium can be achieved. In addition, by repeating the circulation step, the cell proliferation in the first pool P1 and the antibody production in the second pool P2 can be sequentially performed. That is, since the continuous culture can be performed, the production efficiency of the antibody can be improved.

### Industrial Applicability

According to the present disclosure, it is possible to perform culture corresponding to a growth phase of cells in a compact configuration.

### Reference Signs List

1: bioreactor system
10: bioreactor
20: casing
21: lower bottom portion
22: outer cylinder
23: lid portion
23a: through portion
30: inner cylinder
40: rotating unit
41: axis portion
42: upper bottom portion
43: rotating cylinder
43a: lower end edge portion
43b: opening portion
50: drive unit
60: first culture medium supply unit
61: first supply pipe
61a: first supply port
62: first supply line
63: first supply valve
64: first culture medium accommodation unit
70: second culture medium supply unit
71: second supply pipe
71a: second supply port
72: second supply line
73: second supply valve
74: second culture medium accommodation unit
80: cell supply unit
81: cell supply pipe
81a: cell supply port
82: cell supply line
83: cell supply valve
84: cell accommodation unit
91: first temperature adjusting unit
92: second temperature adjusting unit
100: oxygen supply unit
101: first oxygen supply unit
102: second oxygen supply unit
110: removal unit
111: discharge line
112: discharge valve
113: removal pump
120: cell acquisition unit
130: antibody acquisition unit
140: circulation line
141: circulation valve
150: transfer mechanism
151: transfer line
152: transfer valve
160: slide door
161: stopper
170: opening and closing door
171: hinge
180: seal portion
181: rotating seal piece
182: fixed seal piece
183: annular groove
184: fin
185: cutout portion
186: fin
190: low thermal conductivity member
200: control device
201: culture medium supply control unit
202: temperature setting unit
203: cell supply control unit
204: culture execution unit
205: transfer execution unit
206: discharge execution unit
207: circulation execution unit
208: determination unit
211: valve control unit
212: oxygen supply control unit
213: rotation control unit
214: pump control unit
301: CPU
302: main memory
303: storage
304: interface
O: axis line
P1: first pool
P2: second pool

## Claims

1. A bioreactor comprising:
an outer cylinder centered on an axis line extending in a vertical direction;
an inner cylinder provided inside the outer cylinder around the axis line;
a rotating cylinder disposed between the outer cylinder and the inner cylinder to partition a first pool between the rotating cylinder and the inner cylinder, and a second pool between the rotating cylinder and the outer cylinder and rotatable about the axis line;
a drive unit configured to rotationally drive the rotating cylinder about the axis line;
a first supply port configured to supply liquid to the first pool; and
a second supply port configured to supply liquid to the second pool.

2. The bioreactor according to claim 1, further comprising:
a first culture medium supply unit configured to supply, through the first supply port, a first culture medium as the liquid to the first pool; and
a second culture medium supply unit configured to supply, through the second supply port, a second culture medium having a component different from the first culture medium as the liquid to the second pool.

3. The bioreactor according to claim 1, further comprising:
a first temperature adjusting unit configured to adjust a temperature of the first pool; and
a second temperature adjusting unit configured to adjust a temperature of the second pool independently of the first temperature adjusting unit.

4. The bioreactor according to claim 1, further comprising:
an oxygen supply unit configured to supply oxygen to the first pool and the second pool.

5. The bioreactor according to claim 4,
wherein the oxygen supply unit includes
a first oxygen supply unit configured to supply oxygen to the first pool, and
a second oxygen supply unit configured to supply oxygen to the second pool independently of the first oxygen supply unit.

6. The bioreactor according to any one of claims 1 to 5, further comprising:
a cell supply unit configured to supply cells to the first pool; and
a transfer mechanism configured to transfer at least a part of the liquid in the first pool to the second pool.

7. The bioreactor according to claim 6,
wherein the transfer mechanism includes
a transfer tube connecting the first pool and the second pool, and
a transfer valve provided in the transfer tube and configured to be opened and closed.

8. The bioreactor according to claim 6,
wherein the drive unit is configured to rotationally drive the rotating cylinder in forward and reverse directions,
the rotating cylinder has an opening portion that penetrates the rotating cylinder in a radial direction, and
the transfer mechanism includes an opening portion and closing portion configured to close the opening portion when the rotating cylinder rotates in the forward direction and to open the opening portion when the rotating cylinder rotates in the reverse direction.

9. The bioreactor according to claim 8,
wherein the transfer mechanism includes a slide door that is slidable between a closing position in which the opening portion is closed, and an opening position located on a forward side of the closing position in a forward rotation direction, in which the opening portion is opened.

10. The bioreactor according to claim 8,
wherein the transfer mechanism includes
an opening and closing door provided on a forward side in the forward rotation direction of the opening portion to be rotatable about a rotation axis line extending in a vertical direction, and
the opening and closing door is rotatable between a closing position in which the opening portion is closed, and an opening position that is separated from the opening portion to open the opening portion.

11. The bioreactor according to any one of claims 1 to 5, further comprising:
a removal unit including a discharge line configured to allow liquid in the second pool to be removed to the outside, a cell acquisition unit provided in the discharge line and extracting cells from the liquid, and an antibody acquisition unit provided in the discharge line and extracting an antibody produced by the cells from the liquid.

12. The bioreactor according to claim 11,
wherein the discharge line passes through an inside of the inner cylinder, and
at least one of the cell acquisition unit and the antibody acquisition unit is provided inside the inner cylinder.

13. A bioreactor according to claim 11,
wherein the removal unit includes a circulation line connected on a downstream side of the cell acquisition unit and the antibody acquisition unit in the discharge line, the circulation line being configured to return the liquid to the second pool.

14. The bioreactor according to any one of claims 1 to 5, further comprising:
a lower bottom portion closing the outer cylinder from below,
wherein a lower end edge portion of the rotating cylinder faces an upper surface of the bottom portion through a clearance.

15. The bioreactor according to claim 14, further comprising:
a seal portion sealing between the lower end edge portion of the rotating cylinder and the upper surface of the lower bottom portion.

16. A bioreactor system comprising:
the bioreactor according to claim 1; and
a control device that controls the bioreactor,
wherein the bioreactor includes
a first culture medium supply unit configured to supply, through the first supply port, a first culture medium as the liquid to the first pool, and
a second culture medium supply unit configured to supply, through the second supply port, a second culture medium having a component different from the first culture medium as the liquid to the second pool, and
the control device includes
a culture medium supply control unit performing a culture medium supply step in which the first culture medium is supplied to the first pool by controlling the first culture medium supply unit, and the second culture medium is supplied to the second pool by controlling the second culture medium supply unit.

17. A method for operating a bioreactor including an outer cylinder centered on an axis line extending in a vertical direction, an inner cylinder provided inside the outer cylinder around the axis line, a rotating cylinder disposed between the outer cylinder and the inner cylinder to partition a first pool between the rotating cylinder and the inner cylinder, and a second pool between the rotating cylinder and the outer cylinder and rotatable about the axis line, and a drive unit configured to rotationally drive the rotating cylinder about the axis line, the method comprising:
a culture medium supply step of supplying a first culture medium to the first pool and supplying a second culture medium having a component different from the first culture medium to the second pool.

18. The method for operating a bioreactor according to claim 17, further comprising:
a cell supply step of supplying cells to the first pool after the culture medium supply step; and
a culture step of rotationally driving the rotating cylinder via the drive unit after the cell supply step.

19. The method for operating a bioreactor according to claim 18, further comprising:
a transfer step of transferring liquid in the first pool to the second pool after the culture step,
wherein the culture step is performed again after the transfer step.

20. The method for operating a bioreactor according to claim 19, further comprising:
a discharge step of removing the liquid from the second pool after completion of the culture step after the transfer step, and extracting the cells and an antibody produced by the cells from the liquid.
